# EUROPEAN PATENT APPLICATION

(11) **EP 4 201 930 A1**
(43) Date of publication of application: **28.06.2023**
(21) Application number: 21856230.4
(22) Date of filing: 11.08.2021
(51) Int. Cl.: C07D 405/04, C07D 409/14, C07D 405/10, C07D 491/048, C07D 495/04, H01L 51/00, H01L 51/50

(54) **HETEROCYCLIC COMPOUND, ORGANIC LIGHT-EMITTING DEVICE COMPRISING SAME, AND COMPOSITION FOR ORGANIC MATERIAL LAYER OF ORGANIC LIGHT-EMITTING DEVICE**

(30) Priority: 13.08.2020 KR 20200101857
(71) Applicant: LT Materials Co., Ltd., Yongin-si, Gyeonggi-do 17118 (KR)
(72) Inventor: LEE, Sol, Yongin-si, Gyeonggi-do 17118 (KR); MO, Jun-Tae, Yongin-si, Gyeonggi-do 17118 (KR); BYUN, Ji-Yoon, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/010661
(87) International publication number: WO 2022/035224

(57) **Abstract**

The present application provides a heterocyclic compound capable of significantly enhancing lifetime, efficiency, electrochemical stability and thermal stability of an organic light emitting device, an organic light emitting device comprising the heterocyclic compound in an organic material layer, and a composition for an organic material layer.

## Description

### [Technical Field]

This application claims priority to and the benefits of Korean Patent Application No. 10-2020-0101857, filed with the Korean Intellectual Property Office on August 13, 2020, the entire contents of which are incorporated herein by reference.

The present specification relates to a heterocyclic compound, an organic light emitting device comprising the same, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a heterocyclic compound, an organic light emitting device comprising the same, and a composition for an organic material layer of an organic light emitting device.

### [Technical Solution]

One embodiment of the present application provides a heterocyclic compound represented by the following Chemical Formula 1.

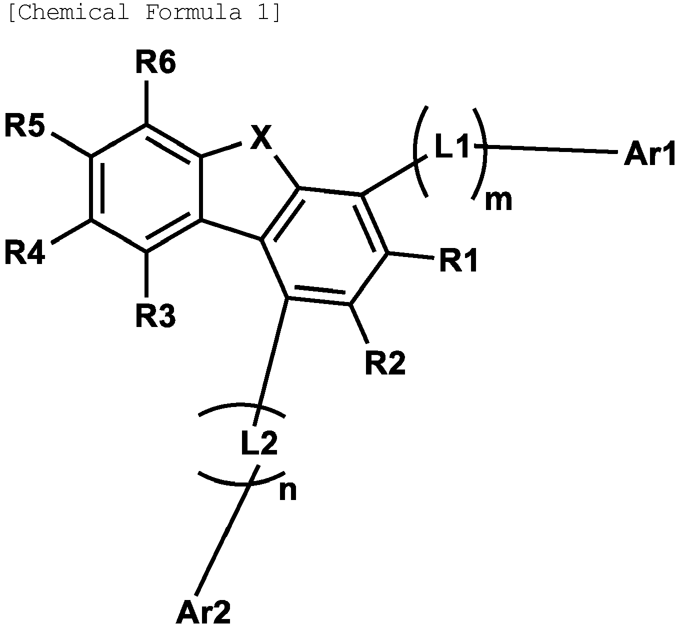

In Chemical Formula 1,
X is O; or S,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms,
R1 to R6 are the same as or different from each other, and each independently hydrogen; deuterium; a substituted or unsubstituted aryl group having 6 to 60 carbon atoms; or a substituted or unsubstituted heteroaryl group having 2 to 60 carbon atoms,
Ar1 and Ar2 are the same as or different from each other, and each independently a monocyclic or polycyclic heterocyclic group having 2 to 60 carbon atoms substituted or unsubstituted and comprising one or more 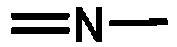 bonds; or a substituted or unsubstituted amine group, and
m and n are each an integer of 0 to 3, and when m and n are each 2 or greater, substituents in the parentheses are the same as or different from each other.

In addition, another embodiment of the present application provides an organic light emitting device comprising a first electrode, a second electrode, and one or more organic material layers provided between the first electrode and the second electrode, wherein one or more layers of the organic material layers comprise the heterocyclic compound represented by Chemical Formula 1.

Another embodiment of the present application provides a composition for an organic material layer of an organic light emitting device, the composition comprising the heterocyclic compound represented by Chemical Formula 1 and one of heterocyclic compounds represented by the following Chemical Formulae 4 to 6.

### [Advantageous Effects]

A heterocyclic compound according to one embodiment of the present application can be used as a material of an organic material layer of an organic light emitting device. The heterocyclic compound can be used as a material of a hole injection layer, a hole transfer layer, a light emitting layer, an electron transfer layer, an electron injection layer, a charge generation layer and the like in an organic light emitting device. Particularly, the heterocyclic compound represented by Chemical Formula 1 can be used as a material of a light emitting layer of an organic light emitting device. In addition, using the heterocyclic compound represented by Chemical Formula 1 in an organic light emitting device is capable of lowering a driving voltage of the device, enhancing light efficiency, and enhancing lifetime properties of the device by thermal stability of the compound.

### [Description of Drawings]

FIG. 1 to FIG. 3 are diagrams each schematically illustrating a lamination structure of an organic light emitting device according to one embodiment of the present application.
FIG. 4 is a diagram showing changes in PL (photoluminescence) when using compounds of the present application as a single host according to an example.
FIG. 5 is a diagram showing a change in PL (photoluminescence) when using compounds of the present application as a mixed host according to an example.

### [Mode for Disclosure]

Hereinafter, the present application will be described in detail.

One embodiment of the present application provides a heterocyclic compound represented by the following Chemical Formula 1.

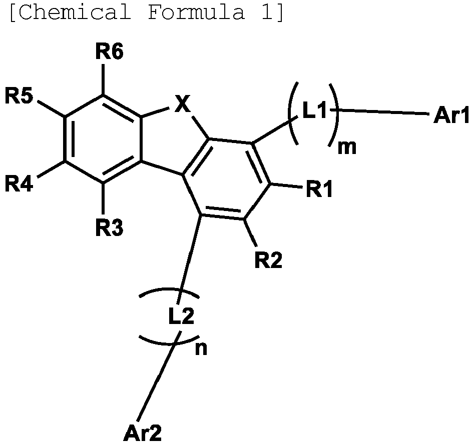

In Chemical Formula 1
X is O; or S,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms,
R1 to R6 are the same as or different from each other, and each independently hydrogen; deuterium; a substituted or unsubstituted aryl group having 6 to 60 carbon atoms; or a substituted or unsubstituted heteroaryl group having 2 to 60 carbon atoms,
Ar1 and Ar2 are the same as or different from each other, and each independently a monocyclic or polycyclic heterocyclic group having 2 to 60 carbon atoms substituted or unsubstituted and comprising one or more 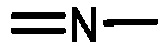 bonds; or a substituted or unsubstituted amine group, and
m and n are each an integer of 0 to 3, and when m and n are each 2 or greater, substituents in the parentheses are the same as or different from each other.

By having an amine group and a monocyclic or polycyclic heterocyclic group having 2 to 60 carbon atoms comprising one or more 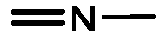 bonds as substituents in the dibenzofuran or dibenzothiophene structure, Chemical Formula 1 is more electron abundant, and, by improving a current flow, an effect of lowering a driving voltage is obtained when using the compound represented by Chemical Formula 1 in a device. In addition, by having an amine group having hole properties as a substituent, Chemical Formula 1 has an excellent hole transfer ability, and an effect of lowering a driving voltage is obtained when using the compound represented by Chemical Formula 1 in a device.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, the "substituted or unsubstituted" means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 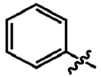 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

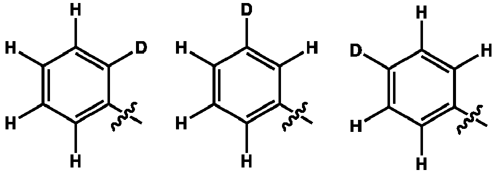

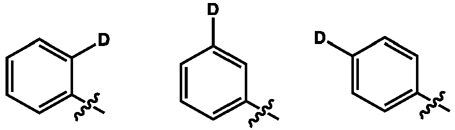

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

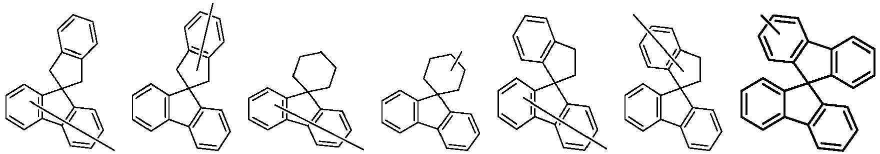

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto. In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

The heterocyclic compound according to one embodiment of the present application is represented by Chemical Formula 1. More specifically, by having a core structure and structural properties of the substituents as above, the heterocyclic compound represented by Chemical Formula 1 may be used as a material of an organic material layer of an organic light emitting device.

In one embodiment of the present application, the heterocyclic compound represented by Chemical Formula 1 may have a deuterium content of 0% to 100%.

In one embodiment of the present application, the heterocyclic compound represented by Chemical Formula 1 may have a deuterium content of greater than or equal to 10% and less than or equal to 100%.

In one embodiment of the present application, the heterocyclic compound represented by Chemical Formula 1 may have a deuterium content of greater than or equal to 20% and less than or equal to 100%.

In one embodiment of the present application, the heterocyclic compound represented by Chemical Formula 1 may have a deuterium content of greater than or equal to 30% and less than or equal to 100%.

In one embodiment of the present application, the heterocyclic compound represented by Chemical Formula 1 may have a deuterium content of greater than or equal to 40% and less than or equal to 100%.

In one embodiment of the present application, L1 and L2 of Chemical Formula 1 are the same as or different from each other, and may be each independently a direct bond; a substituted or unsubstituted arylene group having 6 to 60 carbon atoms; or a substituted or unsubstituted heteroarylene group having 2 to 60 carbon atoms.

In one embodiment of the present application, L1 and L2 are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L1 and L2 are the same as or different from each other, and may be each independently a direct bond; a substituted or unsubstituted arylene group having 6 to 20 carbon atoms; or a substituted or unsubstituted heteroarylene group having 2 to 20 carbon atoms.

In one embodiment of the present application, L1 may be a direct bond; a substituted or unsubstituted arylene group having 6 to 60 carbon atoms; or a substituted or unsubstituted heteroarylene group having 2 to 60 carbon atoms.

In another embodiment, L1 is a direct bond.

In another embodiment, L1 is an arylene group having 6 to 60 carbon atoms; or a substituted or unsubstituted heteroarylene group having 2 to 60 carbon atoms.

In another embodiment, L1 is a phenylene group.

In one embodiment of the present application, L2 may be a direct bond; a substituted or unsubstituted arylene group having 6 to 60 carbon atoms; or a substituted or unsubstituted heteroarylene group having 2 to 60 carbon atoms.

In another embodiment, L2 is a direct bond.

In another embodiment, L2 is an arylene group having 6 to 60 carbon atoms; or a substituted or unsubstituted heteroarylene group having 2 to 60 carbon atoms.

In another embodiment, L2 is a phenylene group.

In one embodiment of the present application, X of Chemical Formula 1 may be O; or S.

In another embodiment, X of Chemical Formula 1 is O.

In another embodiment, X of Chemical Formula 1 is S.

In one embodiment of the present application, R1 to R6 of Chemical Formula 1 are the same as or different from each other, and may be each independently hydrogen; deuterium; a substituted or unsubstituted aryl group having 6 to 60 carbon atoms; or a substituted or unsubstituted heteroaryl group having 2 to 60 carbon atoms.

In one embodiment of the present application, R1 to R6 are the same as or different from each other, and may be each independently a substituted or unsubstituted aryl group having 6 to 60 carbon atoms; or a substituted or unsubstituted heteroaryl group having 2 to 60 carbon atoms.

In one embodiment of the present application, R1 to R6 are the same as or different from each other, and may be each independently hydrogen; or deuterium.

In another embodiment, R1 to R6 are hydrogen.

In another embodiment, R1 to R6 are deuterium.

In one embodiment of the present application, Ar1 and Ar2 of Chemical Formula 1 are the same as or different from each other, and may be each independently a monocyclic or polycyclic heterocyclic group having 2 to 60 carbon atoms substituted or unsubstituted and comprising one or more =N- bonds; or a substituted or unsubstituted amine group.

In one embodiment of the present application, at least one of Ar1 and Ar2 is a substituted or unsubstituted amine group.

In one embodiment of the present application, Ar1 may be a substituted or unsubstituted amine group, and Ar2 may be a monocyclic or polycyclic heterocyclic group having 2 to 60 carbon atoms substituted or unsubstituted and comprising one or more 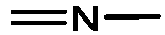 bonds.

In one embodiment of the present application, Ar1 may be an amine group unsubstituted or substituted with one or more selected from the group consisting of an aryl group having 6 to 60 carbon atoms and a heteroaryl group having 2 to 60 carbon atoms, and Ar2 may be a monocyclic or polycyclic heterocyclic group having 2 to 60 carbon atoms substituted or unsubstituted and comprising one or more 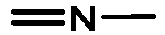 bonds.

In one embodiment of the present application, Ar1 may be an amine group unsubstituted or substituted with one or more selected from the group consisting of an aryl group having 6 to 40 carbon atoms and a heteroaryl group having 2 to 40 carbon atoms, and Ar2 may be a monocyclic or polycyclic heterocyclic group having 2 to 60 carbon atoms substituted or unsubstituted and comprising one or more 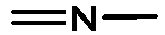 bonds.

In one embodiment of the present application, Ar1 may be an amine group unsubstituted or substituted with one or more selected from the group consisting of an aryl group having 6 to 20 carbon atoms and a heteroaryl group having 2 to 20 carbon atoms, and Ar2 may be a monocyclic or polycyclic heterocyclic group having 2 to 60 carbon atoms substituted or unsubstituted and comprising one or more 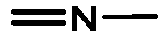 bonds.

In one embodiment of the present application, Ar1 may be an amine group unsubstituted or substituted with one or more selected from the group consisting of a substituted or unsubstituted phenyl group, a substituted or unsubstituted biphenyl group, a substituted or unsubstituted fluorenyl group, a substituted or unsubstituted dibenzofuran group and a substituted or unsubstituted dibenzothiophenyl group, and Ar2 may be a monocyclic or polycyclic heterocyclic group having 2 to 60 carbon atoms substituted or unsubstituted and comprising one or more 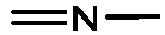 bonds.

In one embodiment of the present application, Ar1 may be an amine group unsubstituted or substituted with one or more selected from the group consisting of a substituted or unsubstituted phenyl group, a substituted or unsubstituted biphenyl group, a fluorenyl group substituted with one or more selected from the group consisting of alkyl groups having 1 to 10 carbon atoms, a substituted or unsubstituted dibenzofuran group and a substituted or unsubstituted dibenzothiophenyl group, and Ar2 may be a monocyclic or polycyclic heterocyclic group having 2 to 60 carbon atoms substituted or unsubstituted and comprising one or more 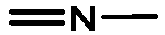 bonds.

In one embodiment of the present application, Ar2 may be an amine group unsubstituted or substituted with one or more selected from the group consisting of an aryl group having 6 to 60 carbon atoms and a heteroaryl group having 2 to 60 carbon atoms, and Ar1 may be a monocyclic or polycyclic heterocyclic group having 2 to 60 carbon atoms substituted or unsubstituted and comprising one or more 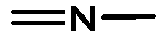 bonds.

In one embodiment of the present application, Ar2 may be an amine group unsubstituted or substituted with one or more selected from the group consisting of an aryl group having 6 to 40 carbon atoms and a heteroaryl group having 2 to 40 carbon atoms, and Ar1 may be a monocyclic or polycyclic heterocyclic group having 2 to 60 carbon atoms substituted or unsubstituted and comprising one or more 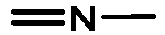 bonds.

In one embodiment of the present application, Ar2 may be an amine group unsubstituted or substituted with one or more selected from the group consisting of an aryl group having 6 to 20 carbon atoms and a heteroaryl group having 2 to 20 carbon atoms, and Ar1 may be a monocyclic or polycyclic heterocyclic group having 2 to 60 carbon atoms substituted or unsubstituted and comprising one or more 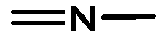 bonds.

In one embodiment of the present application, Ar2 may be an amine group unsubstituted or substituted with one or more selected from the group consisting of a substituted or unsubstituted phenyl group, a substituted or unsubstituted biphenyl group, a substituted or unsubstituted fluorenyl group, a substituted or unsubstituted dibenzofuran group and a substituted or unsubstituted dibenzothiophenyl group, and Ar1 may be a monocyclic or polycyclic heterocyclic group having 2 to 60 carbon atoms substituted or unsubstituted and comprising one or more 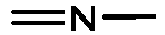 bonds.

In one embodiment of the present application, Ar2 may be an amine group unsubstituted or substituted with one or more selected from the group consisting of a substituted or unsubstituted phenyl group, a substituted or unsubstituted biphenyl group, a fluorenyl group substituted with one or more selected from the group consisting of alkyl groups having 1 to 10 carbon atoms, a substituted or unsubstituted dibenzofuran group and a substituted or unsubstituted dibenzothiophenyl group, and Ar1 may be a monocyclic or polycyclic heterocyclic group having 2 to 60 carbon atoms substituted or unsubstituted and comprising one or more 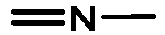 bonds.

In one embodiment of the present application, Chemical Formula 1 may be represented by the following Chemical Formula 2 or 3.

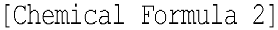

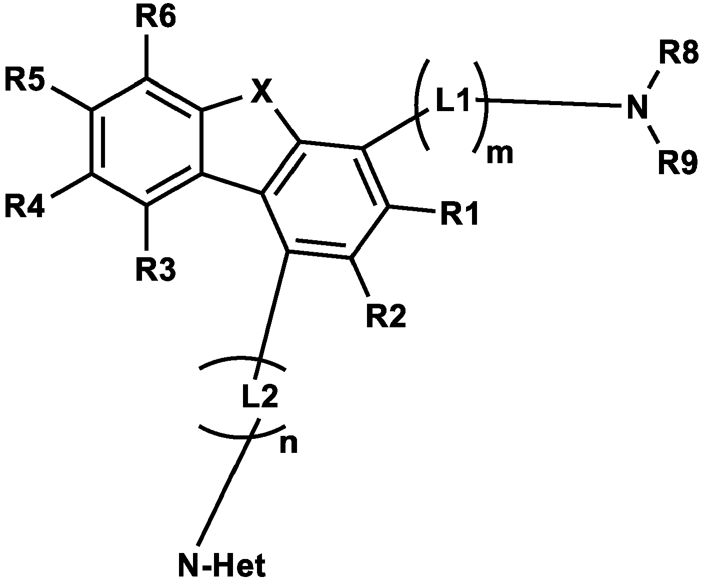

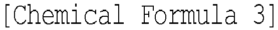

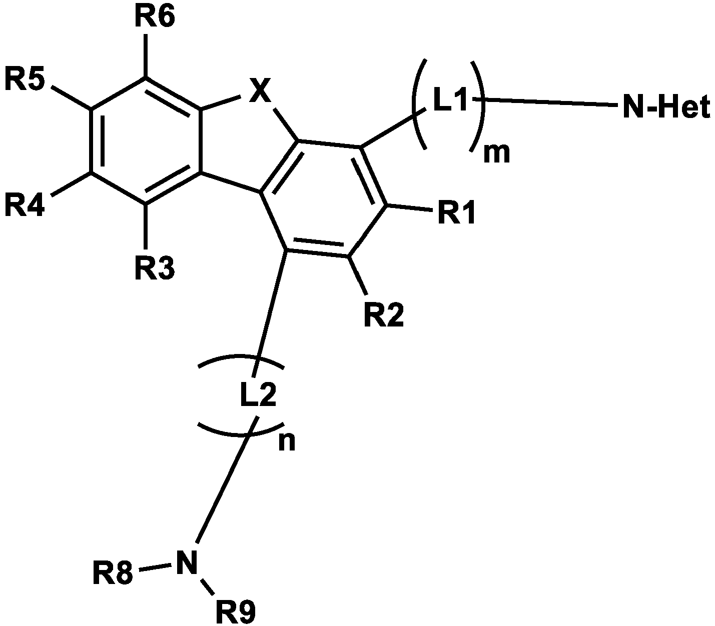

In Chemical Formulae 2 and 3,
R8 and R9 are the same as or different from each other, and each independently hydrogen; deuterium; a substituted or unsubstituted aryl group having 6 to 60 carbon atoms; or a substituted or unsubstituted heteroaryl group having 2 to 60 carbon atoms,
N-Het is a monocyclic or polycyclic heterocyclic group having 2 to 60 carbon atoms substituted or unsubstituted and comprising one or more 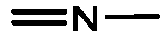 bonds, and
X, L1, L2, R1 to R6, Ar1, Ar2, m and n have the same definitions as in Chemical Formula 1.

When No. 1 and No. 4 positions of dibenzofuran are substituted with an amine group and a monocyclic or polycyclic heterocyclic group having 2 to 60 carbon atoms comprising one or more 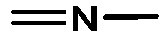 bonds as in Chemical Formulae 2 and 3, it is structurally more stable, and using the heterocyclic compounds represented by Chemical Formula 2 and 3 in an organic light emitting device is effective in further increasing a lifetime of the device, and further lowering a driving voltage of the device due to a decreased band gap.

In the present specification, N-Het of Chemical Formulae 2 and 3 means a monocyclic or polycyclic heterocyclic group having 2 to 60 carbon atoms substituted or unsubstituted and comprising one or more 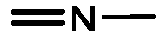 bonds, which Ar1 and Ar2 may become.

In the present specification, "comprising one or more 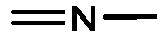 bonds" means comprising one or more double bonds comprising N.

In one embodiment of the present application, R8 and R9 are the same as or different from each other, and may be each independently hydrogen; deuterium; a substituted or unsubstituted aryl group having 6 to 60 carbon atoms; or a substituted or unsubstituted heteroaryl group having 2 to 60 carbon atoms.

In one embodiment of the present application, R8 and R9 are the same as or different from each other, and may be each independently hydrogen; deuterium; a substituted or unsubstituted aryl group having 6 to 40 carbon atoms; or a substituted or unsubstituted heteroaryl group having 2 to 40 carbon atoms.

In one embodiment of the present application, R8 and R9 are the same as or different from each other, and may be each independently hydrogen; deuterium; a substituted or unsubstituted aryl group having 6 to 20 carbon atoms; or a substituted or unsubstituted heteroaryl group having 2 to 20 carbon atoms.

In one embodiment of the present application, R8 and R9 are the same as or different from each other, and may be each independently hydrogen; deuterium; a substituted or unsubstituted heteroaryl group.

In one embodiment of the present application, R8 and R9 are the same as or different from each other, and may be each independently hydrogen; deuterium; a substituted or unsubstituted phenyl group; a substituted or unsubstituted biphenyl group; a substituted or unsubstituted naphthyl group; a substituted or unsubstituted fluorenyl group; a substituted or unsubstituted dibenzofuran group; or a substituted or unsubstituted dibenzothiophenyl group.

In one embodiment of the present application, R8 and R9 are the same as or different from each other, and may be each independently hydrogen; deuterium; a phenyl group; a biphenyl group; a naphthyl group; a fluorenyl group substituted with one or more selected from the group consisting of alkyl groups having 1 to 10 carbon atoms; a dibenzofuran group; or a dibenzothiophenyl group.

In one embodiment of the present application, N-Het may be a group represented by any one of the following Chemical Formulae A-1 to A-3.

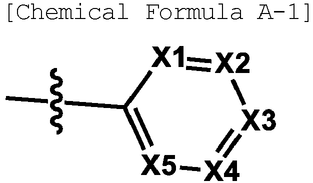

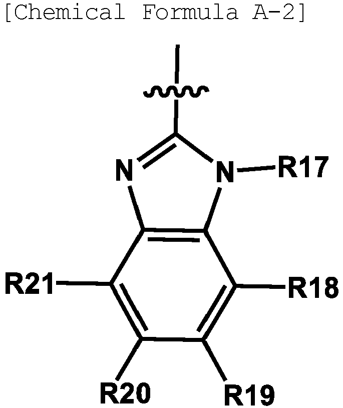

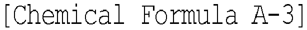

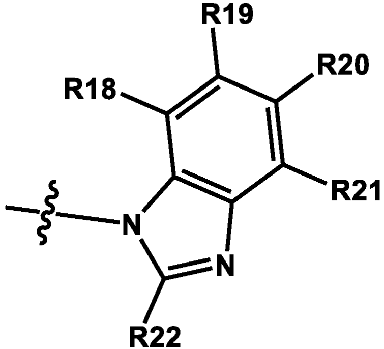

In Chemical Formulae A-1 to A-3,
X1 is CR11 or N, X2 is CR12 or N, X3 is CR13 or N, X4 is CR14 or N, X5 is CR15 or N, and at least one of X1 to X5 is N, and
R11 to R15 and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring, and 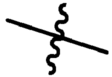 is a site linked to Chemical Formula 1. 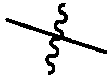 also means a site linked to Chemical Formula 2 or 3.

Since Chemical Formula 1 has arylamine, which has a more favorable hole transfer ability as a donor, that is, has higher donating strength, as a substituent, the balance effect with an acceptor becomes greater resulting in an increased HOMO level compared to having carbazole, which is a donor with weaker donating strength, as a substituent. In other words, using the compound represented by Chemical Formula 1 in a device is effective in further lowering a driving voltage and further increasing a lifetime compared to using a compound having carbazole as a substituent.

In one embodiment of the present application, Chemical Formula A-1 may be represented by the following Group A.

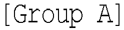

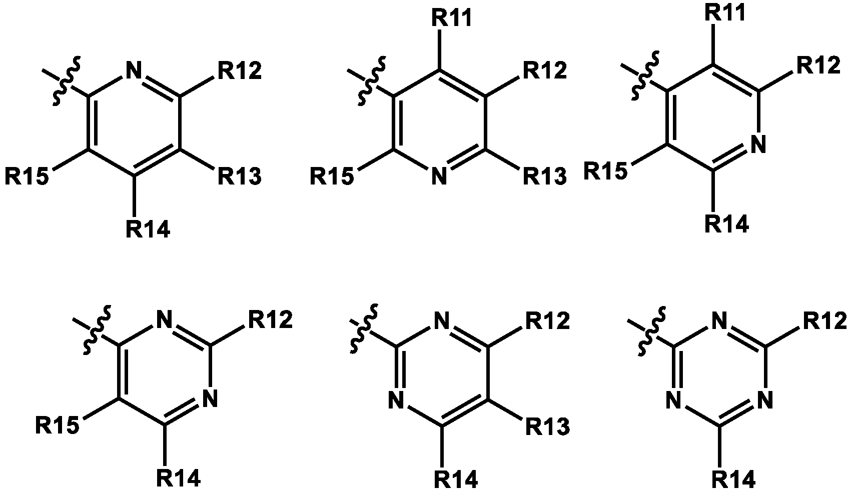

In Group A, R11 to R15 and 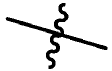 have the same definitions as in Chemical Formula A-1.

In one embodiment of the present application, m and n of Chemical Formula 1 are each an integer of 0 to 3, and when m and n are each 2 or greater, substituents in the parentheses may be the same as or different from each other.

In another embodiment, m is 0.

In another embodiment, m is 1.

In another embodiment, m is 2.

In another embodiment, m is 3.

In one embodiment of the present application, when m is 2 or greater, substituents in the parentheses may be the same as or different from each other.

In another embodiment, n is 0.

In another embodiment, n is 1.

In another embodiment, n is 2.

In another embodiment, n is 3.

In one embodiment of the present application, when n is 2 or greater, substituents in the parentheses may be the same as or different from each other.

According to one embodiment of the present application, Chemical Formula 1 may be represented by any one of the following compounds, but is not limited thereto.

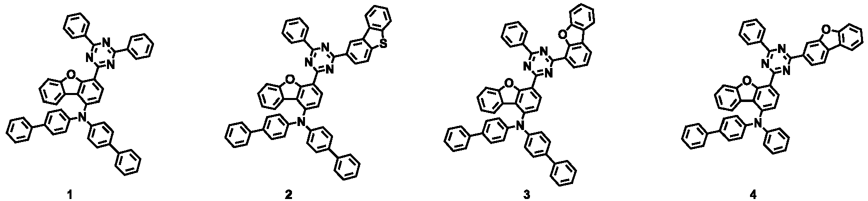

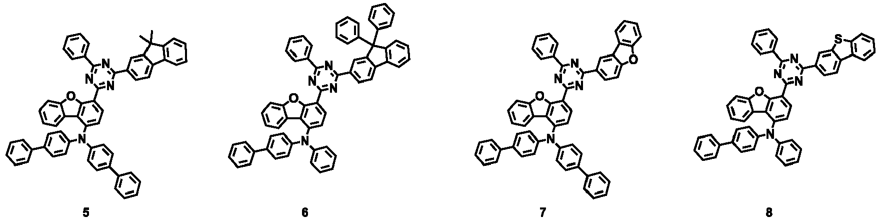

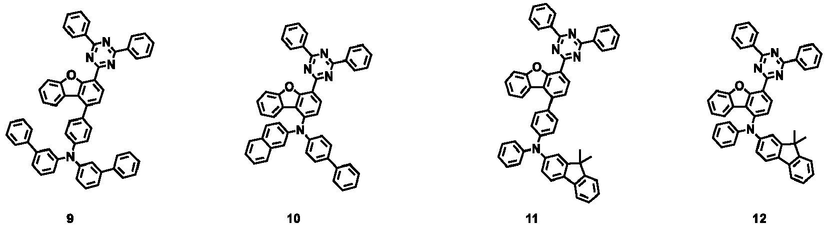

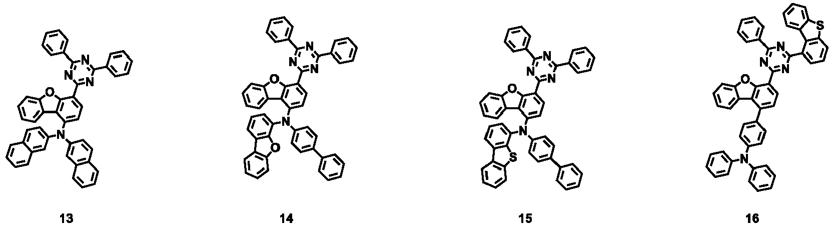

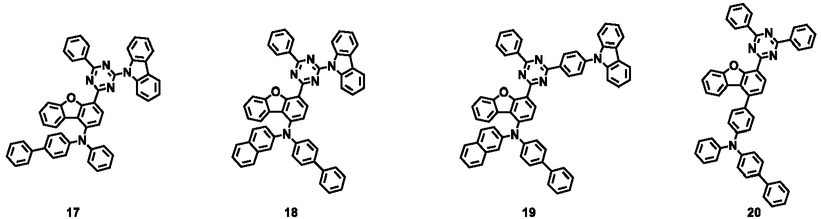

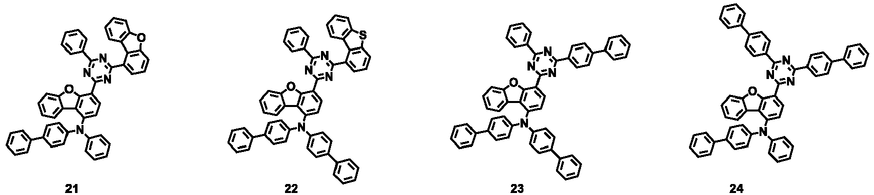

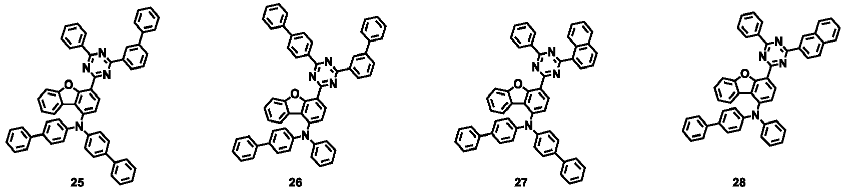

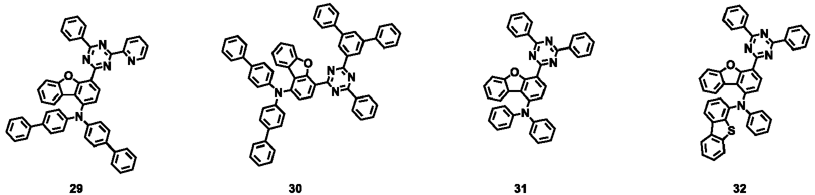

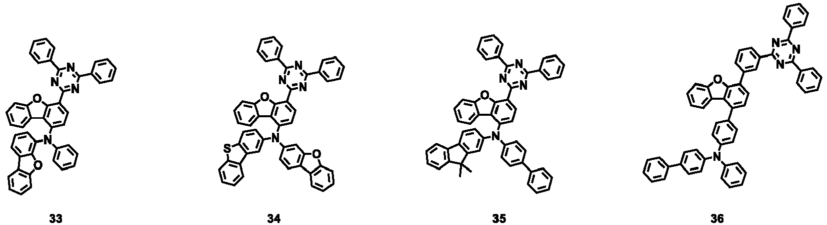

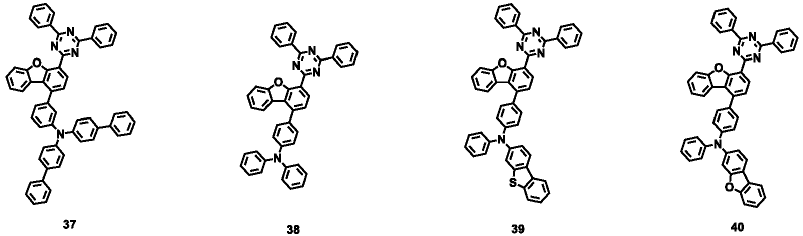

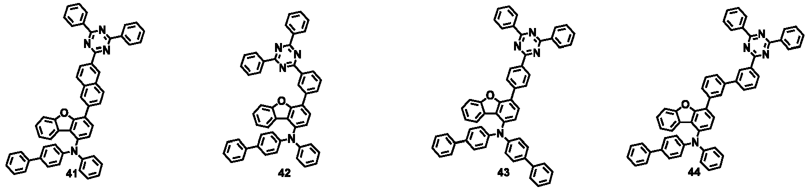

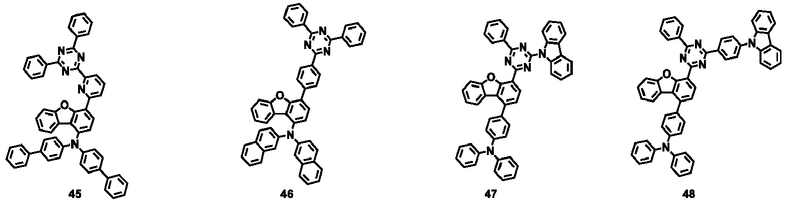

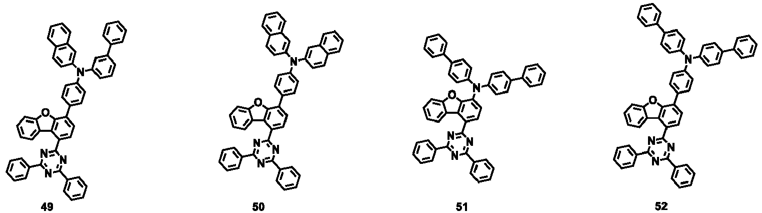

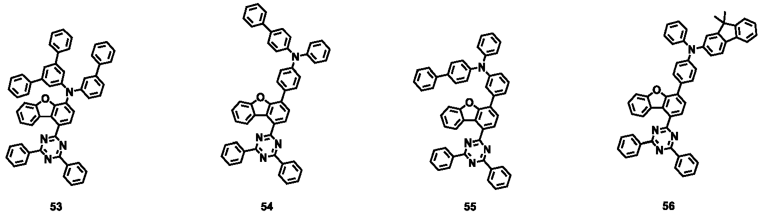

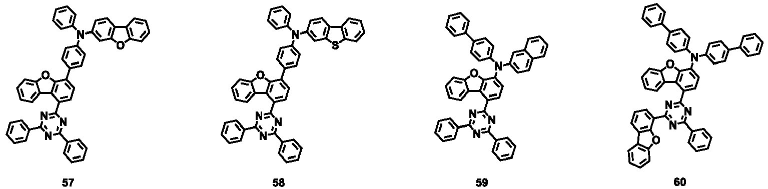

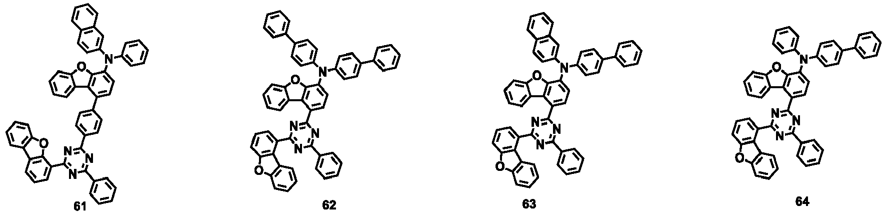

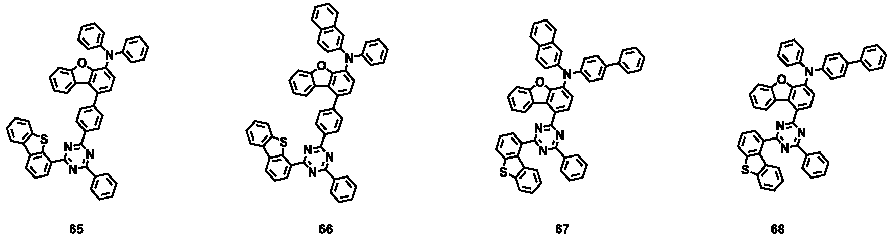

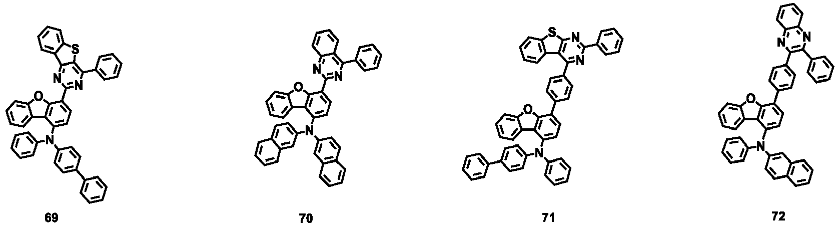

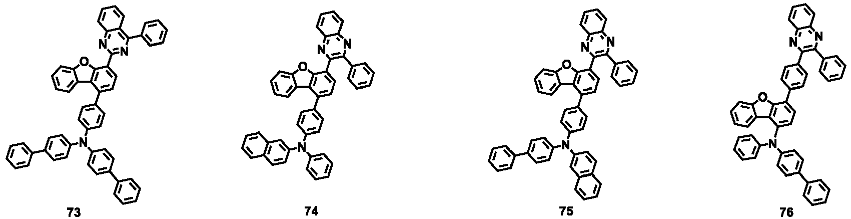

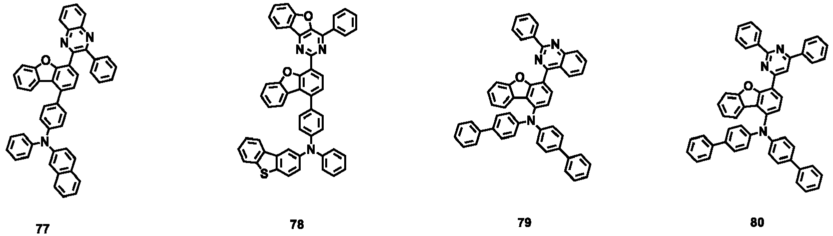

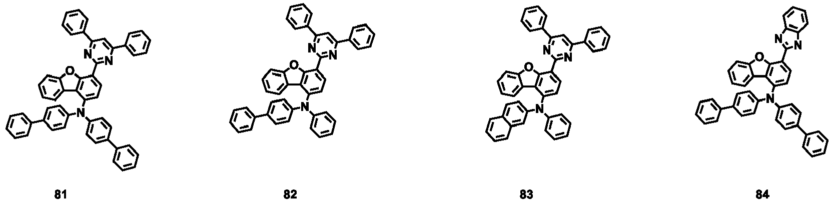

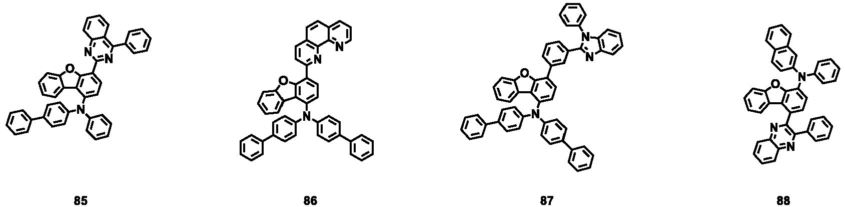

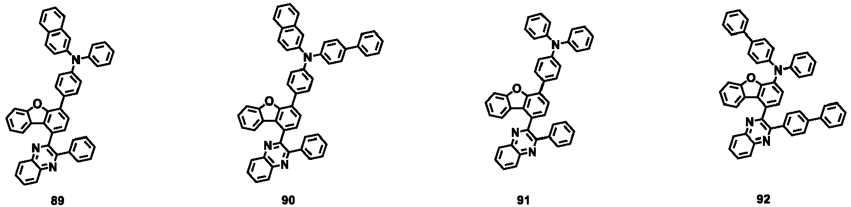

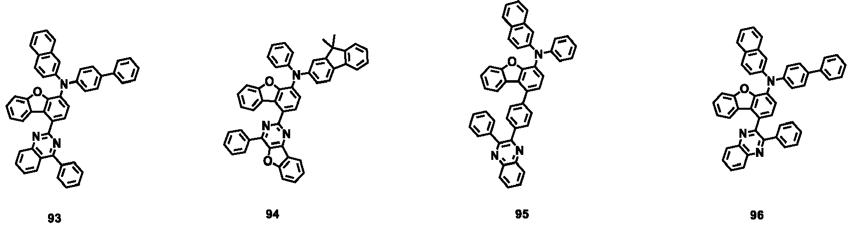

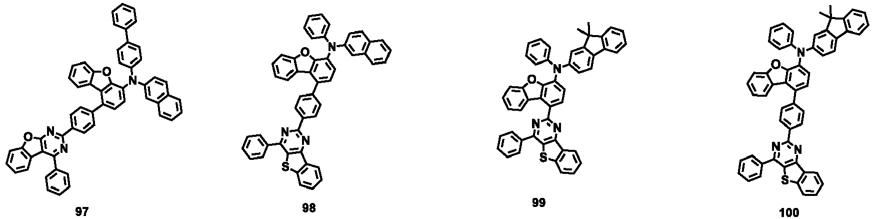

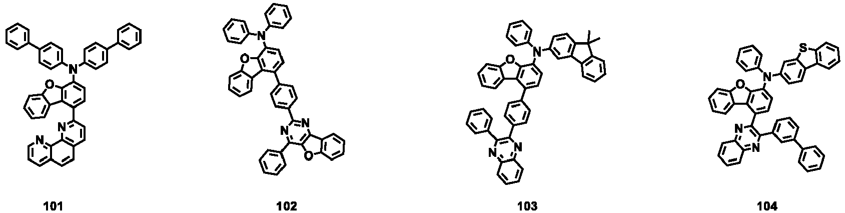

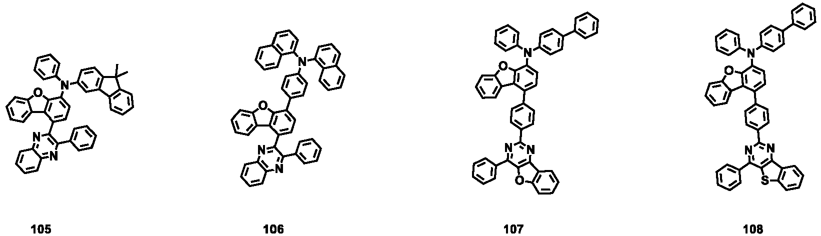

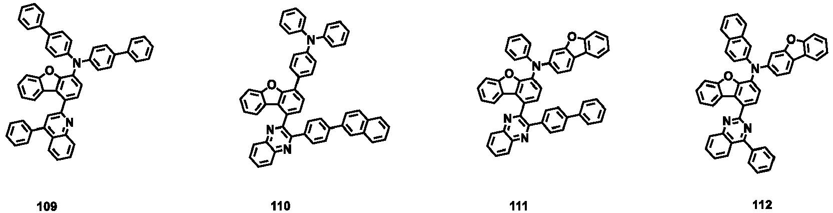

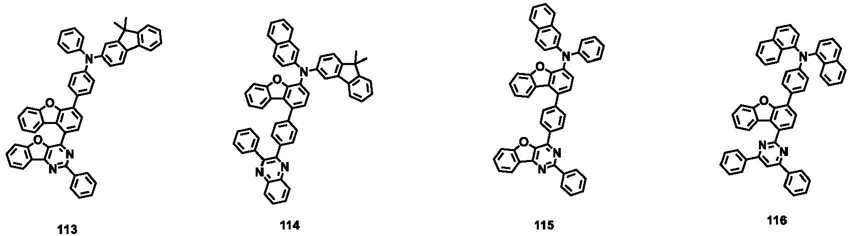

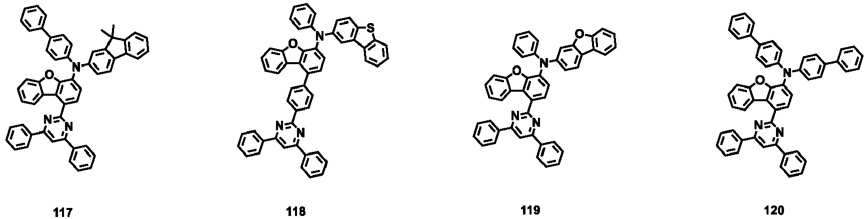

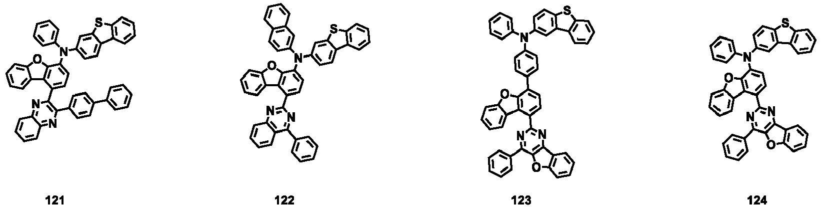

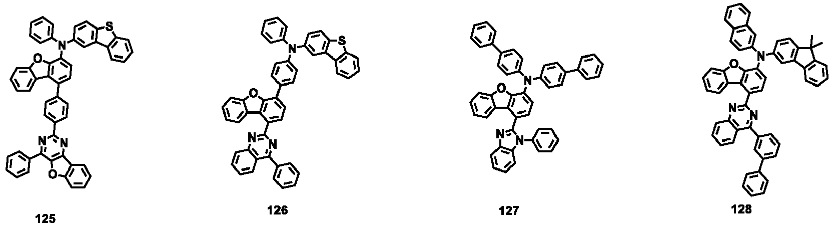

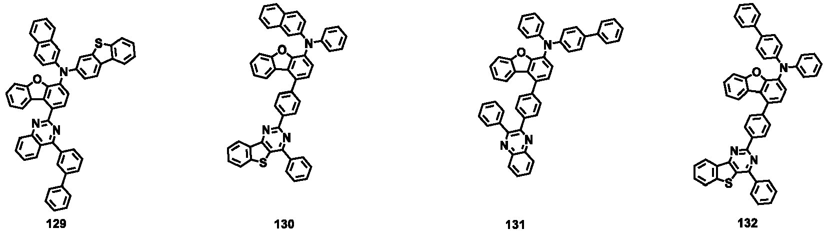

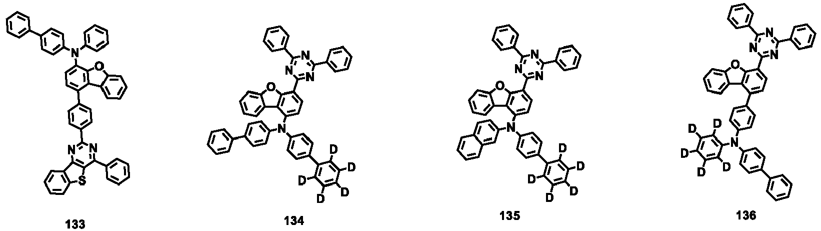

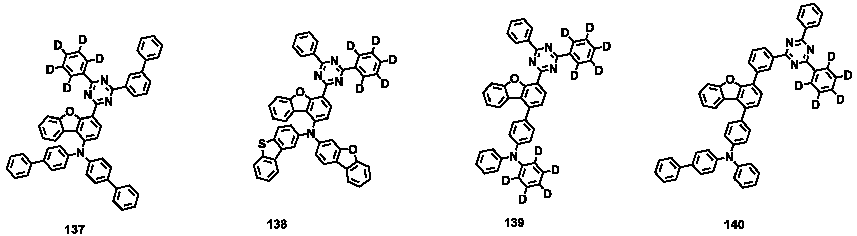

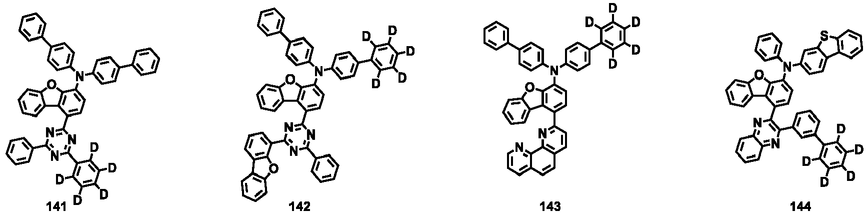

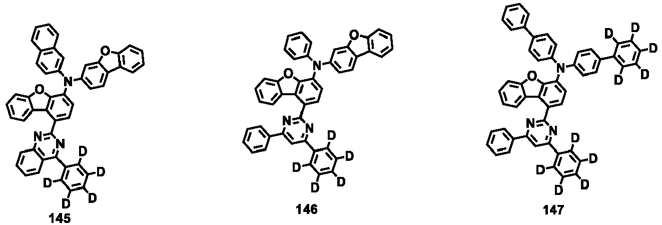

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the compound of Chemical Formula 1 may be prepared. More specifically, the heterocyclic compound according to one embodiment of the present application may be prepared based on preparation examples to describe later.

Another embodiment of the present application provides an organic light emitting device comprising the heterocyclic compound represented by Chemical Formula 1. The "organic light emitting device" may be expressed in terms such as an "organic light emitting diode", an "OLED", an "OLED device" and an "organic electroluminescent device".

The heterocyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises a first electrode, a second electrode, and one or more organic material layers provided between the first electrode and the second electrode, and one or more layers of the organic material layers comprise the heterocyclic compound represented by Chemical Formula 1. When comprising the heterocyclic compound represented by Chemical Formula 1 in the organic material layer, the organic light emitting device has superior light emission efficiency and lifetime.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a red organic light emitting device, and the heterocyclic compound according to Chemical Formula 1 may be used as a material of the red organic light emitting device. The heterocyclic compound according to Chemical Formula 1 has a high HOMO level, and is considered to be more suitable as a red host of an organic light emitting device.

In one embodiment of the present application, the organic light emitting device may be a green organic light emitting device, and the heterocyclic compound according to Chemical Formula 1 may be used as a material of the green organic light emitting device.

In one embodiment of the present application, the organic light emitting device may be a blue organic light emitting device, and the heterocyclic compound according to Chemical Formula 1 may be used as a material of the blue organic light emitting device.

In addition, the organic material layer comprises one or more light emitting layers, and the light emitting layer comprises the heterocyclic compound represented by Chemical Formula 1. When comprising the heterocyclic compound represented by Chemical Formula 1 in the light emitting layer among the organic material layers, the organic light emitting device has more superior light emission efficiency and lifetime.

In addition, in the organic light emitting device of the present application, the organic material layer comprises the heterocyclic compound represented by Chemical Formula 1 as a first compound, and may further comprise one of heterocyclic compounds represented by the following Chemical Formulae 4 to 6 as a second compound.

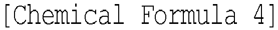

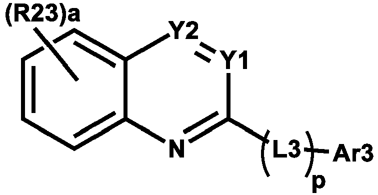

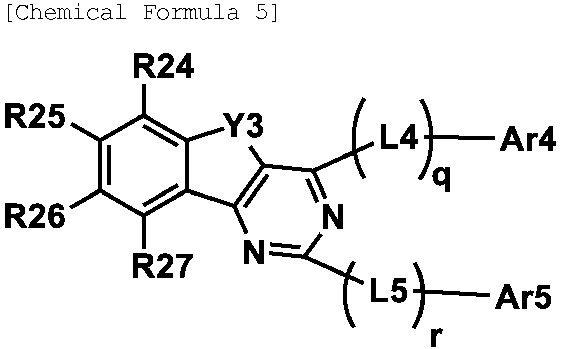

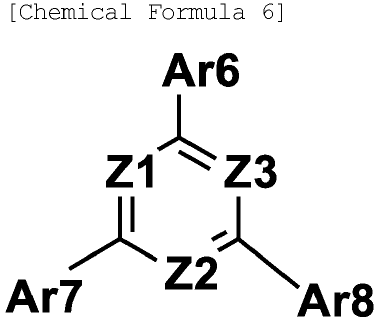

In Chemical Formulae 4 to 6,
L3 to L5 are the same as or different from each other, and each independently a direct bond; a substituted or unsubstituted arylene group having 6 to 60 carbon atoms; or a substituted or unsubstituted heteroarylene group having 2 to 60 carbon atoms,
R23 to R27 are the same as or different from each other, and each independently hydrogen; deuterium; a substituted or unsubstituted aryl group having 6 to 60 carbon atoms; or a substituted or unsubstituted heteroaryl group having 2 to 60 carbon atoms,
Y3 is O; or S,
Ar3 to Ar8 are the same as or different from each other, and each independently a substituted or unsubstituted aryl group having 6 to 60 carbon atoms; or a substituted or unsubstituted heteroaryl group having 2 to 60 carbon atoms,
one of Y1 and Y2 is N, the other one is CRk, and Rk is a substituted or unsubstituted aryl group having 6 to 60 carbon atoms; or a substituted or unsubstituted heteroaryl group having 2 to 60 carbon atoms,
Z1 to Z3 are each independently CH; or N, and at least one of Z1 to Z3 is N,
p, q and r are each an integer of 0 to 3, and when p, q and r are each 2 or greater, substituents in the parentheses are the same as or different from each other, and
a is an integer of 0 to 4, and when a is 2 or greater, substituents in the parentheses are the same as or different from each other.

According to one embodiment of the present application, Chemical Formulae 4 to 6 may be represented by any one of the following compounds, but are not limited thereto.

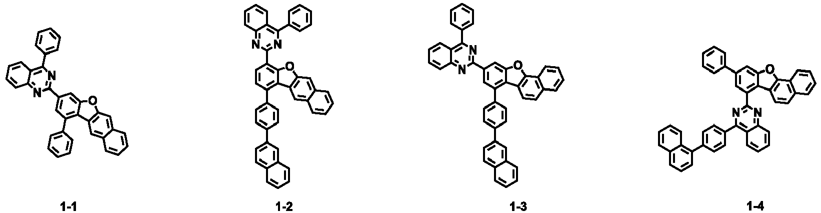

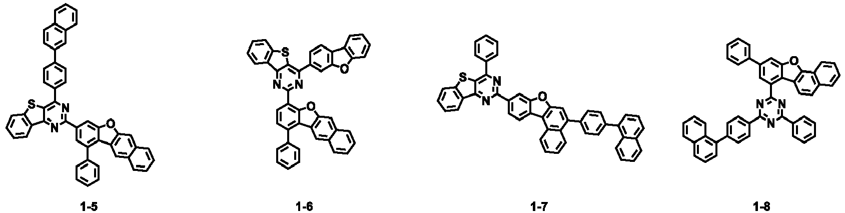

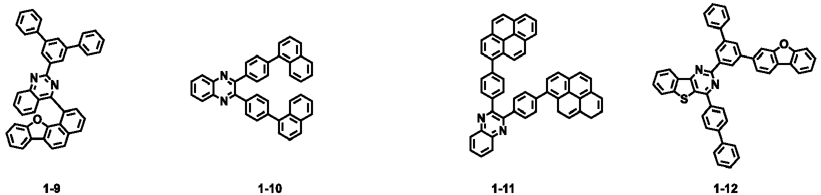

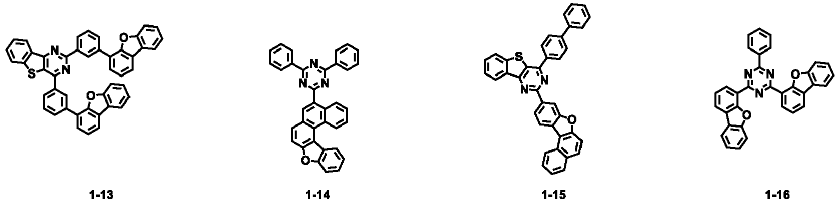

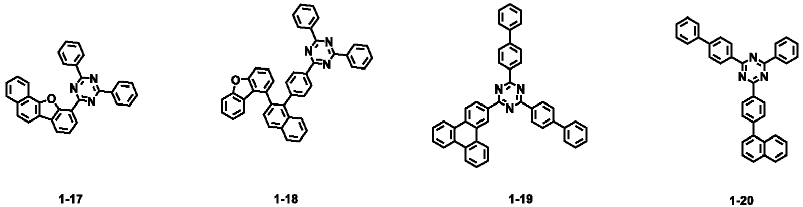

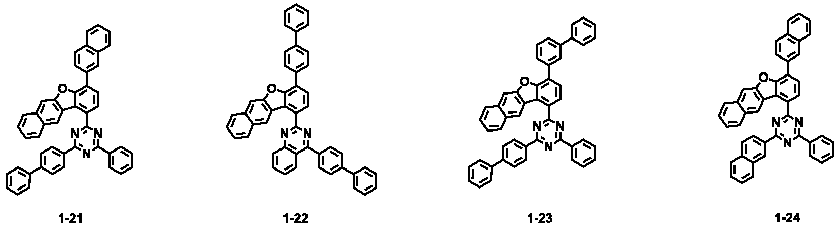

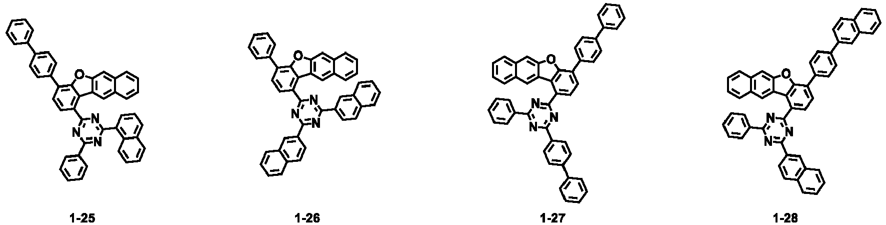

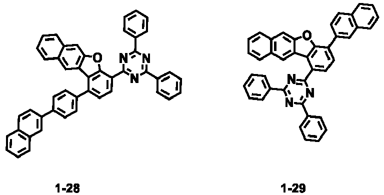

In addition, the organic material layer comprises one or more light emitting layers, and the light emitting layer comprises the heterocyclic compound represented by Chemical Formula 1 as a first compound, and further comprises one of the heterocyclic compounds represented by Chemical Formulae 4 to 6 as a second compound. When comprising the heterocyclic compound represented by Chemical Formula 1 in the light emitting layer among the organic material layers, the organic light emitting device has more superior light emission efficiency and lifetime.

In addition, by introducing various substituents to the structures of Chemical Formulae 4 to 6, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structures of Chemical Formulae 4 to 6, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the compounds of Chemical Formulae 4 to 6 may be prepared. More specifically, the heterocyclic compound according to one embodiment of the present application may be prepared based on preparation examples to describe later.

In addition, the organic material layer comprises one or more light emitting layers, and the light emitting layer further comprises the heterocyclic compound represented by Chemical Formula 1 and one of the heterocyclic compounds represented by Chemical Formulae 4 and 5. When comprising the heterocyclic compound represented by Chemical Formula 1 and one of the heterocyclic compounds represented by Chemical Formulae 4 and 5 in the light emitting layer among the organic material layers, the organic light emitting device has more superior light emission efficiency and lifetime due to an exciplex phenomenon.

In the organic light emitting device of the present application, the organic material layer comprises a light emitting layer, and the light emitting layer may comprise the heterocyclic compound as a host material of a light emitting material.

In the organic light emitting device of the present application, the light emitting layer may comprise two or more host materials, and at least one of the host materials may comprise the heterocyclic compound as a host material of a light emitting material.

In the organic light emitting device of the present application, two or more host materials may be pre-mixed and used as the light emitting layer, and at least one of the two or more host materials may comprise the heterocyclic compound as a host material of a light emitting material.

The pre-mixing means placing and mixing two or more host materials of the light emitting layer in one source of supply before depositing on the organic material layer.

In the organic light emitting device of the present application, the light emitting layer may comprise two or more host materials, and the two or more host materials may each comprise one or more p-type host materials and n-type host materials, and at least one of the host materials may comprise the heterocyclic compound as a host material of a light emitting material. In this case, the organic light emitting device may have superior driving, efficiency and lifetime.

The organic light emitting device of the present disclosure may further comprise one, two or more layers selected from the group consisting of a light emitting layer, a hole injection layer, a hole transfer layer, an electron injection layer, an electron transfer layer, a hole auxiliary layer and a hole blocking layer.

The organic light emitting device according to one embodiment of the present application may be manufactured using common organic light emitting device manufacturing methods and materials except that the organic material layer is formed using the heterocyclic compound described above.

In addition, another embodiment of the present application provides a composition for an organic material layer of an organic light emitting device, the composition comprising the heterocyclic compound represented by Chemical Formula 1 and one of the heterocyclic compounds represented by Chemical Formulae 4 to 6 at the same time.

In another embodiment of the present application, the heterocyclic compound represented by Chemical Formula 1:the heterocyclic compound represented by any one of Chemical Formulae 4 to 6 may have a weight ratio of 1:10 to 10:1, 1:8 to 8:1, 1:5 to 5:1 or 1:2 to 2:1 in the composition, however, the weight ratio is not limited thereto.

Specific descriptions on the heterocyclic compound represented by Chemical Formula 1 and the compounds represented by Chemical Formulae 4 to 6 are the same as the descriptions provided above.

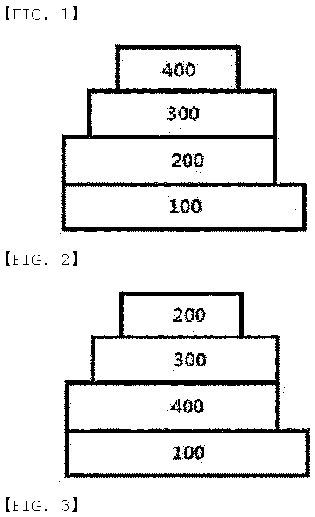

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be comprised, and other necessary functional layers may be further added.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As an example of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Target Compound 1

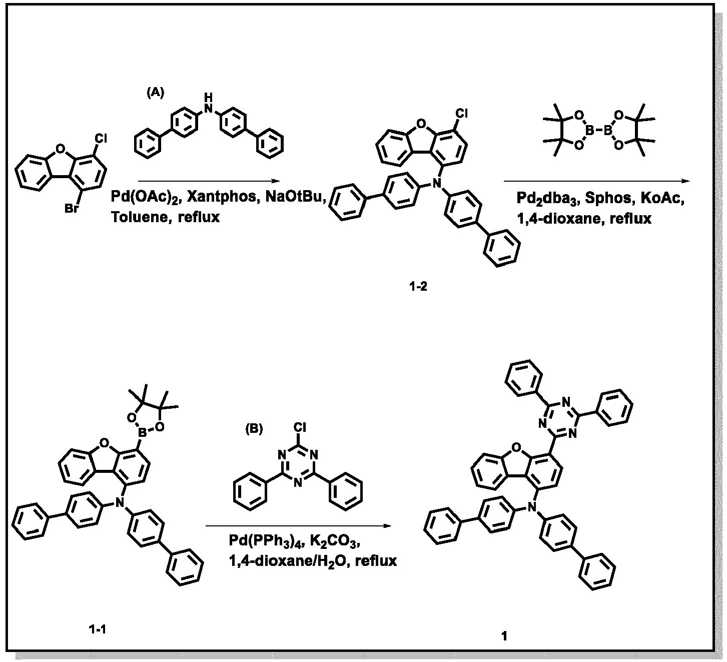

### 1) Preparation of Compound 1-2

1-Bromo-4-chlorodibenzo[b,d]furan (6.0 g, 21.31 mmol), di([1,1'-biphenyl]-4-yl)amine (A) (6.8 g, 21.31 mmol), Pd(OAc)₂ (0.24 g, 1.06 mmol), Xantphos (1.23 g, 2.13 mmol) and NaOtBu (4.1 g, 42.62 mmol) were dissolved in toluene (60 ml), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and the reaction material was purified once again by column chromatography (DCM:Hex=1:5) to obtain Compound 1-2 (5.0 g, 50.35%). The Hex means hexane.

### 2) Preparation of Compound 1-1

Compound 1-2 (5.0 g, 9.58 mmol), bis(pinacolato)diboron (3.16 g, 12.45 mmol), Pd₂(dba)₃ (0.44 g, 1.07 mmol), Sphos (0.4 g, 0.958 mmol) and KOAc (1.9 g, 19.16 mmol) were dissolved in 1,4-dioxane (50 mL), and refluxed for 5 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and then recrystallized with methanol to obtain Compound 1-1 (3.27 g, 56%) .

### 3) Preparation of Target Compound 1

Compound 1-1 (3.27 g, 5.33 mmol), 2-chloro-4,6-diphenyl-1,3,5-triazine (B) (1.43 g, 5.33 mmol), Pd(PPh₃)₄ (0.31 g, 0.27 mmol) and K₂CO₃ (1.47 g, 10.66 mmol) were dissolved in 1,4-dioxane/H₂O (25 mL/5 mL), and refluxed for 3 hours. After the reaction was completed, produced solids were filtered, washed with distilled water, and dried. The dried solids were dissolved in chloroform for silica purification, and the solvent was removed using a rotary evaporator. The result was recrystallized with acetone to obtain target Compound 1 (3.5 g, 91%) .

### 4) Preparation of Additional Target Compounds

Target compounds of the following Table 1 were additionally synthesized in the same manner as in Preparation Example 1 except that A and B of the following Table 1 were used as intermediates instead of using di([1,1'-biphenyl]-4-yl)amine (A) and 2-chloro-4,6-diphenyl-1,3,5-triazine (B) as Intermediates A and B.

**[Table 1]**

| Compo und No. | Intermediate A | Intermediate B | Target Compound | Yield |
|---|---|---|---|---|
| 1 | 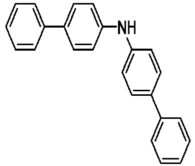 | 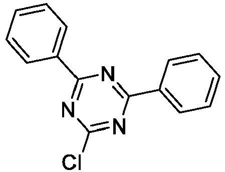 | 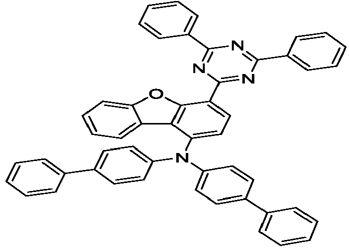 | 50% |
| 4 | 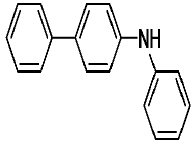 | 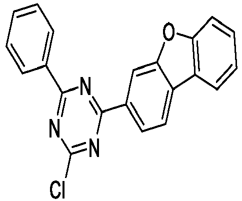 | 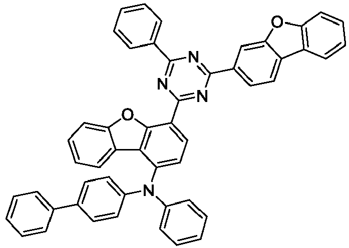 | 57% |
| 12 | 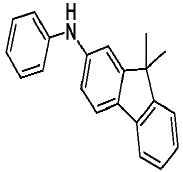 | 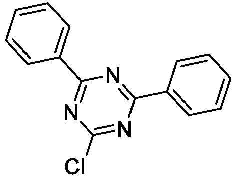 | 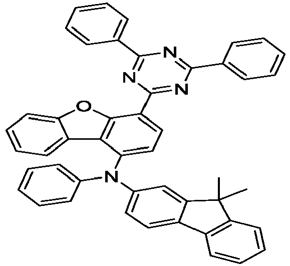 | 70% |
| 14 | 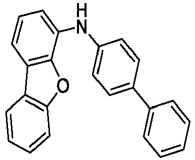 | 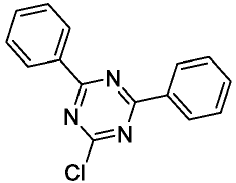 | 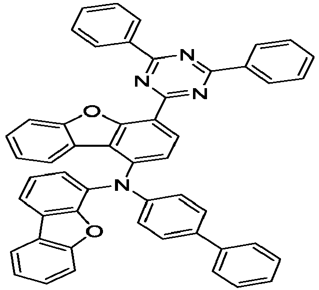 | 65% |
| 15 | 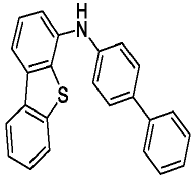 | 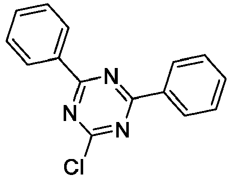 | 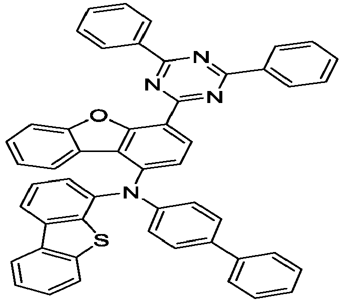 | 59% |
| 21 | 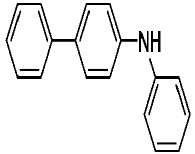 | 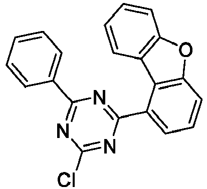 | 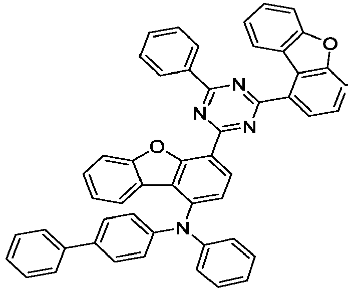 | 55% |
| 27 | 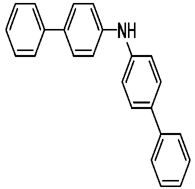 | 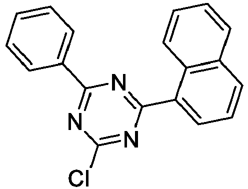 | 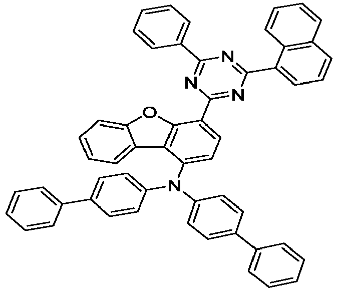 | 66% |
| 28 | 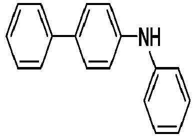 | 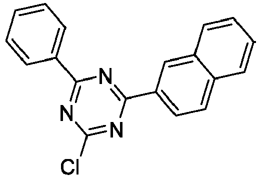 | 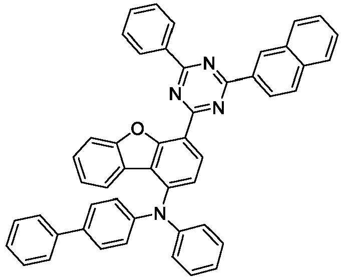 | 55% |
| 32 | 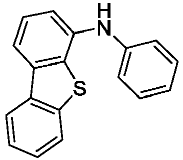 | 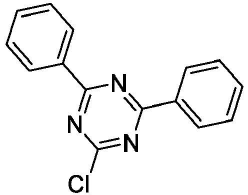 | 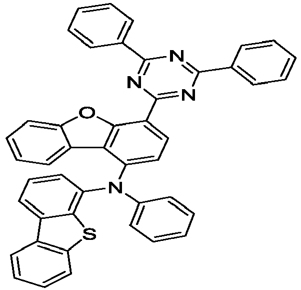 | 45% |
| 33 | 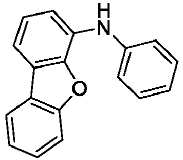 | 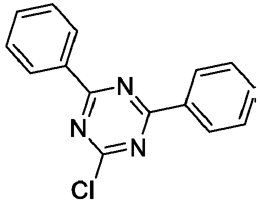 | 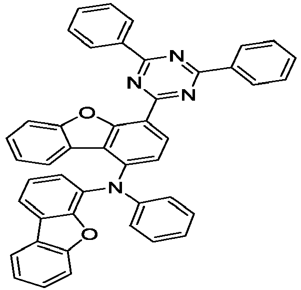 | 59% |
| 35 | 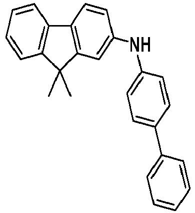 | 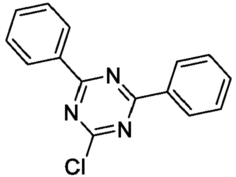 | 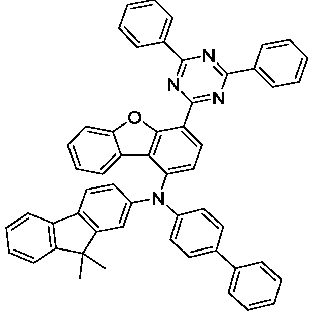 | 66% |
| 69 | 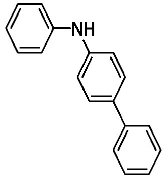 | 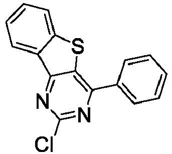 | 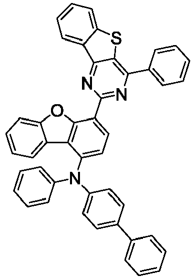 | 57% |
| 80 | 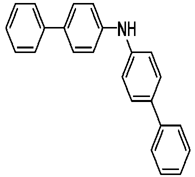 | 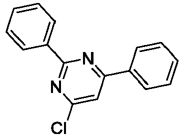 | 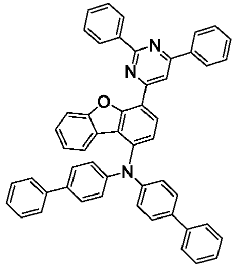 | 62% |
| 81 | 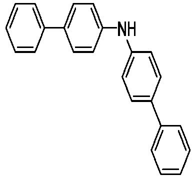 | 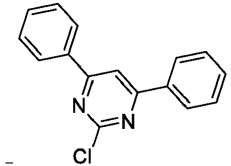 | 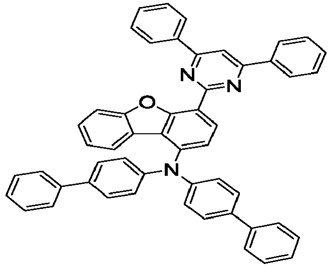 | 45% |
| 82 | 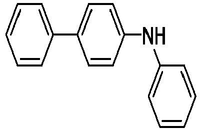 | 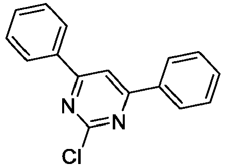 | 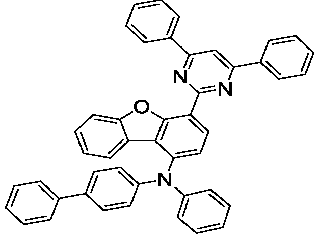 | 51% |

### <Preparation Example 2> Preparation of Target Compound 20

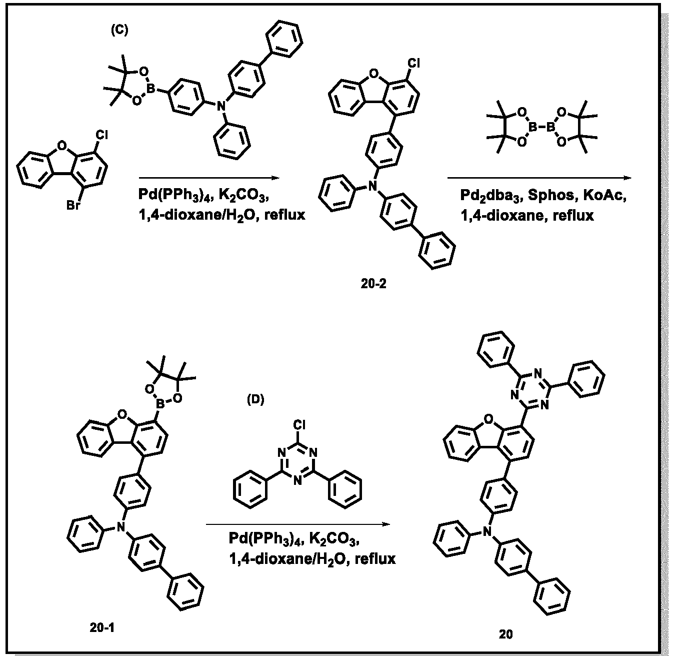

### 1) Preparation of Compound 20-2

1-Bromo-4-chlorodibenzo[b,d]furan (6.0 g, 21.31 mmol), N-phenyl-N-[4-(4,4,5,5-tetramethyl-1,3,2-dioxaborolan-2-yl)phenyl]-[1,1'-biphenyl]-4-amine (C) (9.5 g, 21.31 mmol), Pd(PPh₃)₄ (1.23 g, 1.07 mmol) and K₂CO₃ (5.89 g, 42.62 mmol) were dissolved in 1,4-dioxane/H₂O (30 mL/6 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and the reaction material was purified once again by column chromatography (DCM:Hex=1:5) to obtain Compound 20-2 (5 g, 45%). The Hex means hexane.

### 2) Preparation of Compound 20-1

Compound 20-2 (5.0 g, 9.58 mmol), bis(pinacolato)diboron (3.16 g, 12.45 mmol), Pd₂(dba)₃ (0.44 g, 1.07 mmol), Sphos (0.4 g, 0.958 mmol) and KOAc (1.9 g, 19.16 mmol) were dissolved in 1,4-dioxane (50 mL), and refluxed for 5 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and then recrystallized with methanol to obtain Compound 20-1 (3.27 g, 56%) .

### 3) Preparation of Target Compound 20

Compound 20-1 (3.27 g, 5.33 mmol), 2-chloro-4,6-diphenyl-1,3,5-triazine (D) (1.43 g, 5.33 mmol), Pd(PPh₃)₄ (0.31 g, 0.27 mmol) and K₂CO₃ (1.47 g, 10.66 mmol) were dissolved in 1,4-dioxane/H₂O (25 mL/5 mL), and refluxed for 3 hours. After the reaction was completed, produced solids were filtered, washed with distilled water, and dried. The dried solids were dissolved in chloroform for silica purification, and the solvent was removed using a rotary evaporator. The result was recrystallized with acetone to obtain target Compound 20 (3.5 g, 91%).

### 4) Preparation of Additional Target Compounds

Target compounds of the following Table 2 were additionally synthesized in the same manner as in Preparation Example 2 except that C and D of the following Table 2 were used as intermediates instead of using N-phenyl-N-[4-(4,4,5,5-tetramethyl-1,3,2-dioxaborolan-2-yl)phenyl]-[1,1'-biphenyl]-4-amine (C) and 2-chloro-4,6-diphenyl-1,3,5-triazine (D) as Intermediates C and D.

**[Table 2]**

| Compou nd No. | Intermediate C | Intermediate D | Target Compound | Yield |
|---|---|---|---|---|
| 11 | 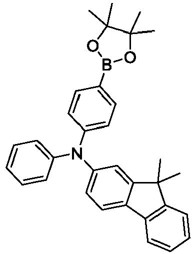 | 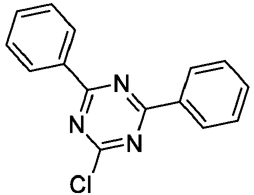 | 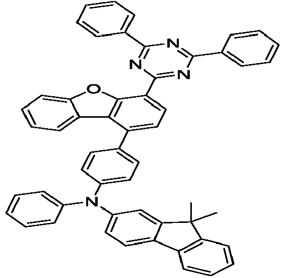 | 50% |
| 16 | 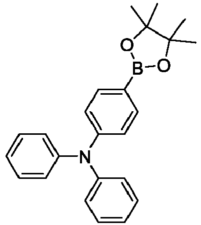 | 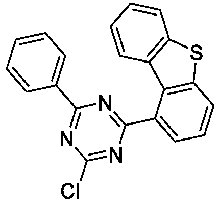 | 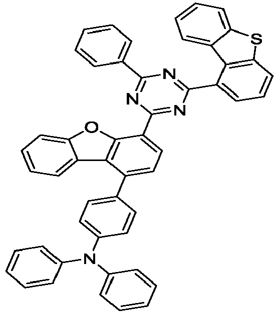 | 57% |
| 20 | 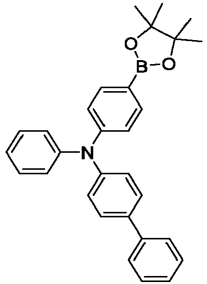 | 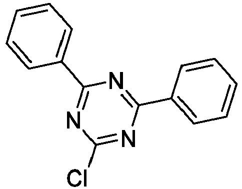 | 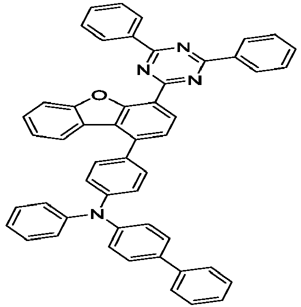 | 70% |
| 39 | 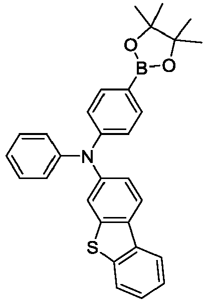 | 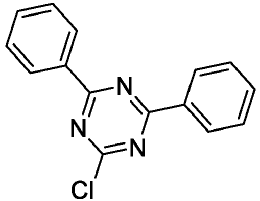 | 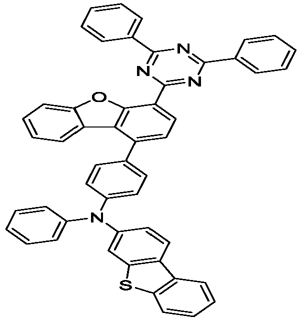 | 65% |
| 40 | 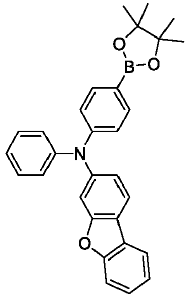 | 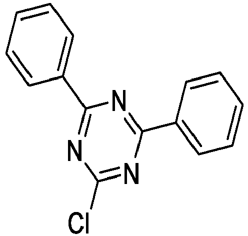 | 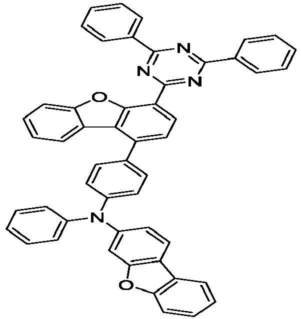 | 59% |
| 47 | 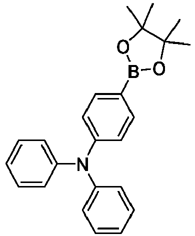 | 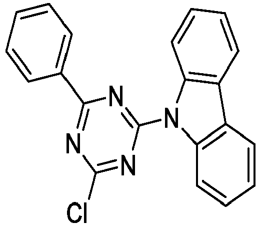 | 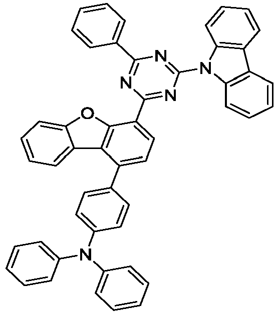 | 55% |
| 74 | 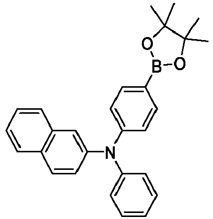 | 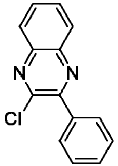 | 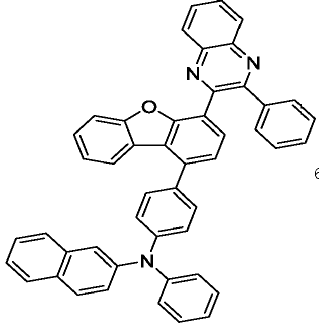 | 66% |
| 78 | 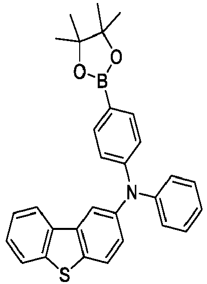 | 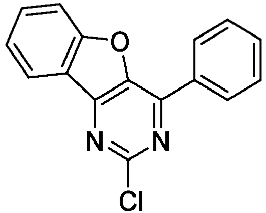 | 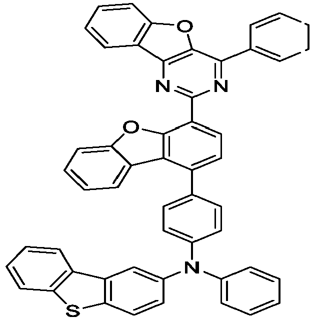 | 68% |
| 136 | 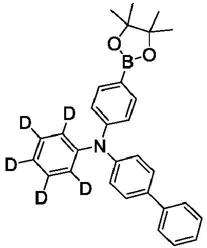 | 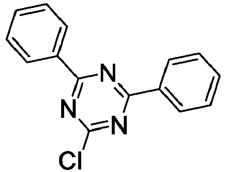 | 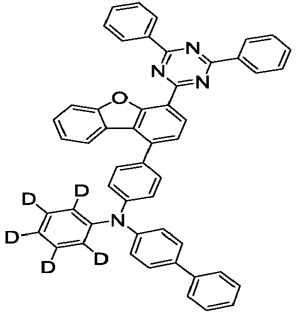 | 44% |
| 139 | 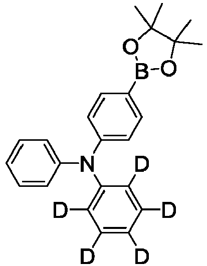 | 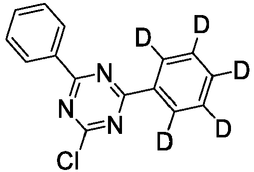 | 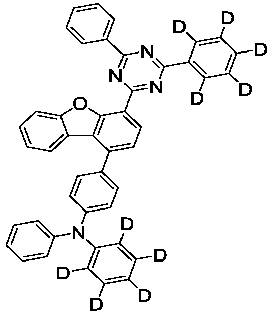 | 58% |

### <Preparation Example 3> Preparation of Target Compound 54

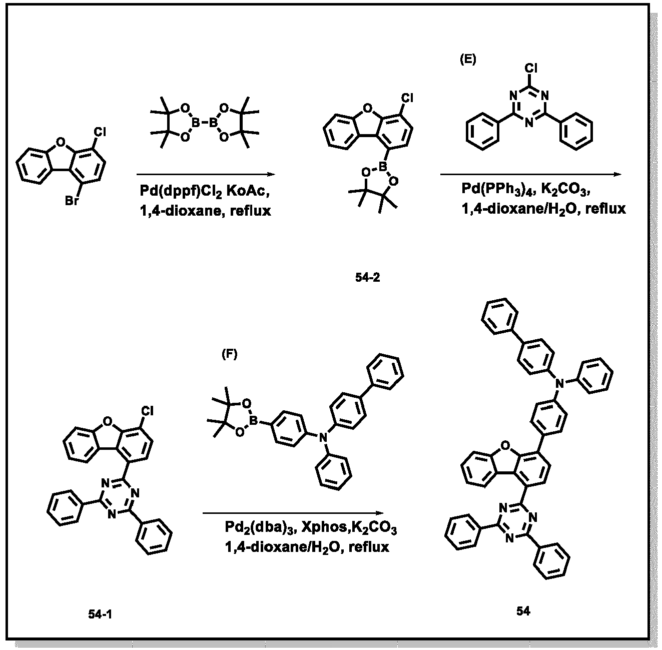

### 1) Preparation of Compound 54-2

1-Bromo-4-chlorodibenzo[b,d]furan (10.0 g, 35.5 mmol), bis(pinacolato)diboron (9.92 g, 39.05 mmol), Pd(dppf)Cl₂ (1.29 g, 1.77 mmol) and KoAc (6.97 g, 71 mmol) were dissolved in 1,4-dioxane (100 mL), and refluxed for 2 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified to obtain Compound 54-2 (10.05 g, 86%).

### 2) Preparation of Compound 54-1

Compound 54-2 (10.05 g 30.58 mmol), 2-chloro-4,6-diphenyl-1,3,5-triazine (E) (8.19 g 30.58 mmol), Pd(PPh₃)₄ (1.77 g 1.53 mmol) and K₂CO₃ (8.45 g, 61.16 mmol) were dissolved in 1,4-dioxane/H₂O (100 mL/20 mL), and refluxed for 4 hours. After the reaction was completed, produced solids were filtered, washed with distilled water, and dried. The dried reaction material was silica purified, and then recrystallized with methanol to obtain Compound 54-1 (11.51 g, 87%).

### 3) Preparation of Target Compound 54

Compound 54-1 (4.69 g 11.43 mmol), N-phenyl-N-(4-(4,4,5,5-tetramethyl-1,3,2-dioxaborolan-2-yl)phenyl)-[1,1'-biphenyl]-4-amine (F) (6.58 g 12.57 mmol), Pd₂(dba)₃ (0.52 g, 0.57 mmol), Xphos (0.545 g, 1.143 mmol) and K₂CO₃ (3.16 g, 22.86 mmol) were dissolved in 1,4-dioxane/H₂O (50 mL/10 mL), and refluxed for 4 hours. After the reaction was completed, produced solids were filtered, washed with distilled water, and dried. The dried solids were dissolved in chloroform for silica purification, and the solvent was removed using a rotary evaporator. The solvent-removed reaction material was silica purified, and the reaction material was purified once again by column chromatography (DCM:Hex=1:5) to obtain target Compound 54 (4.84 g, 53.30%). The Hex means hexane.

### 4) Preparation of Additional Target Compounds

Target compounds of the following Table 3 were additionally synthesized in the same manner as in Preparation Example 3 except that E and F of the following Table 3 were used as intermediates instead of using 2-chloro-4,6-diphenyl-1,3,5-triazine (E) and N-phenyl-N-(4-(4,4,5,5-tetramethyl-1,3,2-dioxaborolan-2-yl)phenyl)-[1,1¹-biphenyl]-4-amine (F) as Intermediates E and F.

**[Table 3]**

| Compoun d No. | Intermediate E | Intermediate F | Target Compound | Yield |
|---|---|---|---|---|
| 52 | 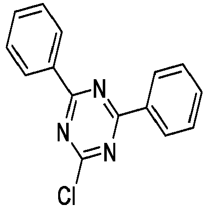 | 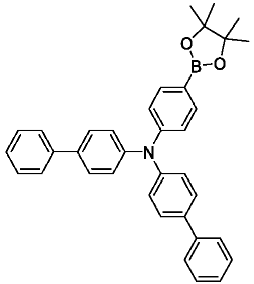 | 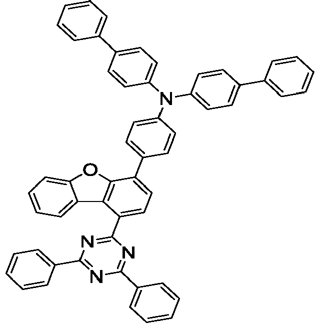 | 50% |
| 54 | 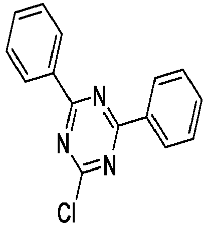 | 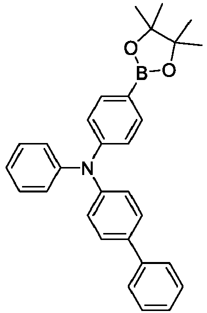 | 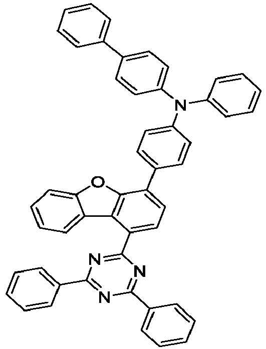 | 57% |
| 55 | 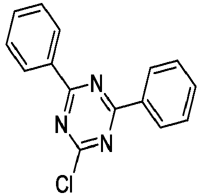 | 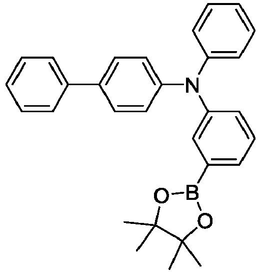 | 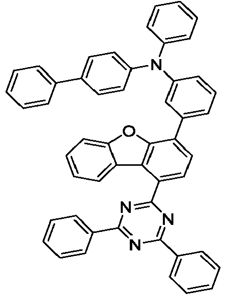 | 70% |
| 58 | 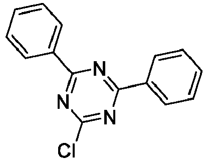 | 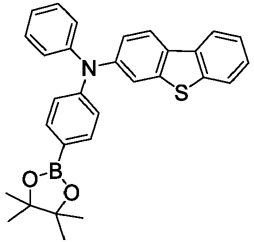 | 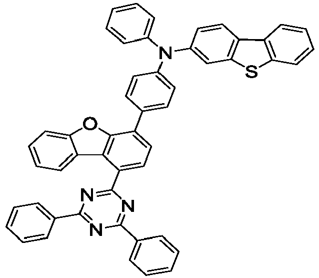 | 65% |
| 89 | 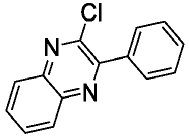 | 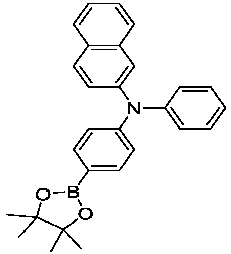 | 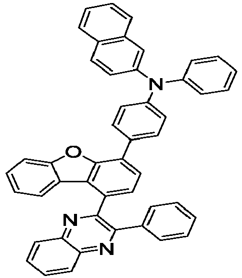 | 59% |
| 113 | 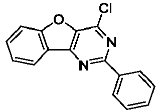 | 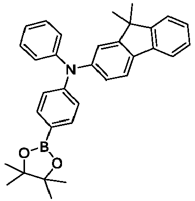 | 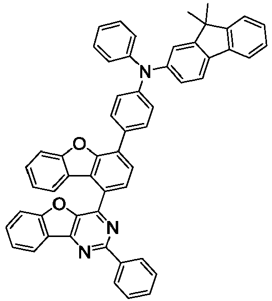 | 66% |
| 116 | 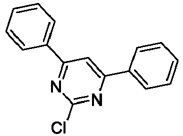 | 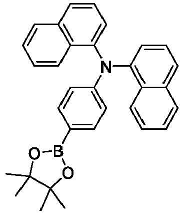 | 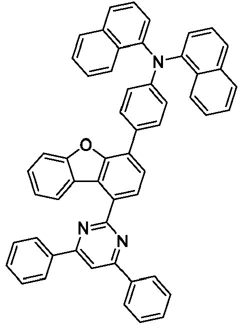 | 52% |
| 123 | 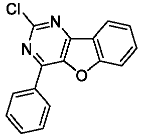 | 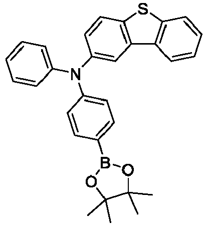 | 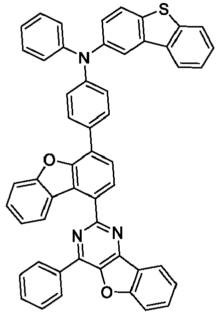 | 48% |
| 126 | 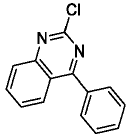 | 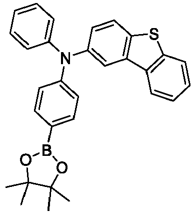 | 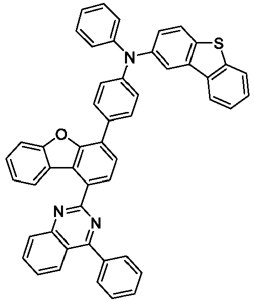 | 57% |

### <Preparation Example 4> Preparation of Target Compound 51

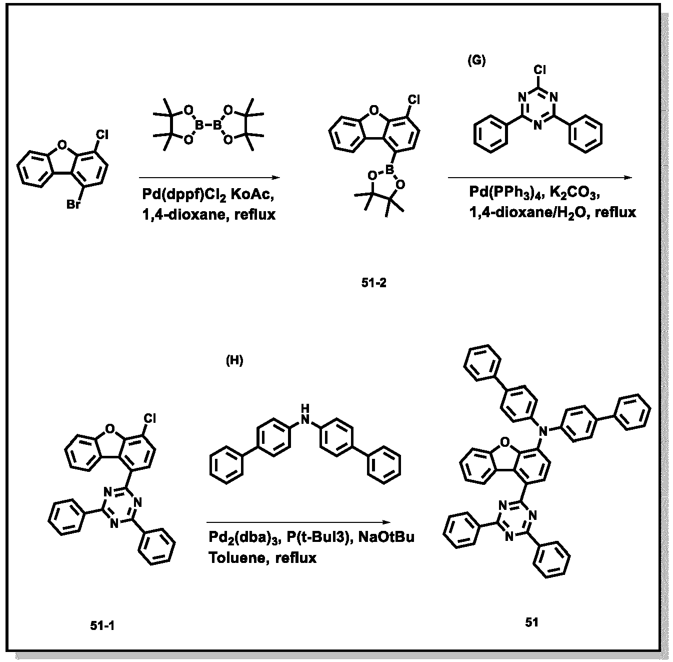

### 1) Preparation of Compound 51-2

1-Bromo-4-chlorodibenzo[b,d]furan (10.0 g, 35.5 mmol), bis(pinacolato)diboron (9.92 g, 39.0 mmol), Pd(dppf)Cl₂ (1.29 g, 1.77 mmol) and KoAc (6.97 g, 71 mmol) were dissolved in 1,4-dioxane (100 mL), and refluxed for 2 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified to obtain Compound 51-2 (10.05 g, 86%).

### 2) Preparation of Compound 51-1

Compound 51-2 (10.05 g, 30.58 mmol), 2-chloro-4,6-diphenyl-1,3,5-triazine (G) (8.19 g, 30.58 mmol), Pd(PPh₃)₄ (1.77 g, 1.53 mmol) and K₂CO₃ (8.45 g, 61.16 mmol) were dissolved in 1,4-dioxane/H₂O (100 mL/20 mL), and refluxed for 4 hours. After the reaction was completed, produced solids were filtered, washed with distilled water, and dried. The reaction material was silica purified, and then recrystallized with methanol to obtain Compound 51-1 (11.51 g, 87%).

### 3) Preparation of Target Compound 51

Compound 51-1 (4.8 g, 11.06 mmol), di([1,1'-biphenyl]-4-yl) amine (H) (4.27 g, 13.27 mmol), Pd₂dba₃ (0.5 g, 0.55 mmol), P(t-Bu)₃ (0.45 g, 1.1 mmol) and NaOtBu (2.13 g, 22.1 mmol) were dissolved in toluene (50 mL), and refluxed for 2 hours. After the reaction was completed, the result was silica purified, and the reaction material was purified by column chromatography (THF:Hex=1:8) to obtain target Compound 51 (3.86 g, 48%). The THF means tetrahydrofuran, and the Hex means hexane.

### 4) Preparation of Additional Target Compounds

Target compounds of the following Table 4 were additionally synthesized in the same manner as in Preparation Example 4 except that G and H of the following Table 4 were used as intermediates instead of using 2-chloro-4,6-diphenyl-1,3,5-triazine (G) and di([1,1'-biphenyl]-4-yl)amine (H) as Intermediates G and H.

**[Table 4]**

| Compoun d No. | Intermediate G | Intermediate H | Target Compound | Yield |
|---|---|---|---|---|
| 51 | 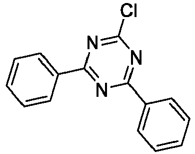 | 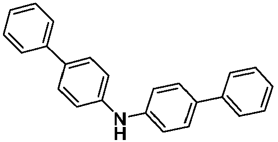 | 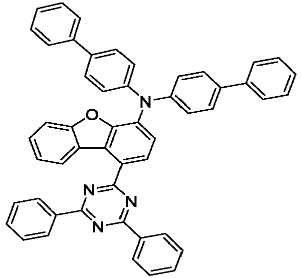 | 50% |
| 60 | 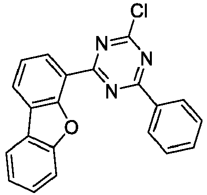 | 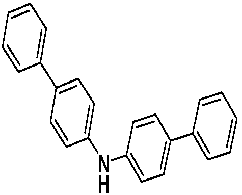 | 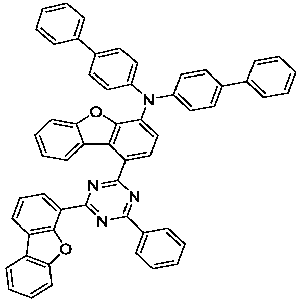 | 57% |
| 64 | 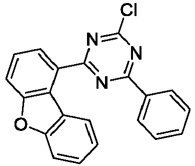 | 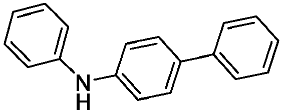 | 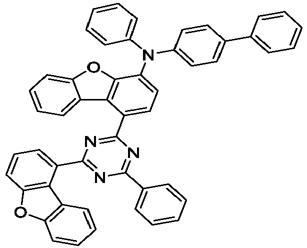 | 70% |
| 67 | 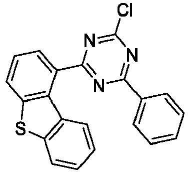 | 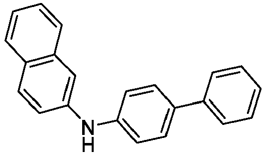 | 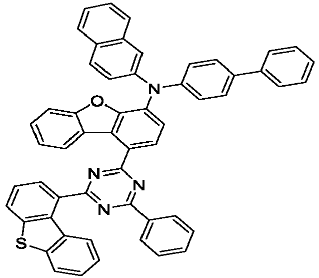 | 65% |
| 88 | 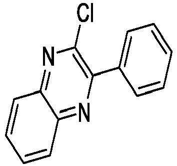 | 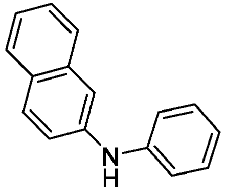 | 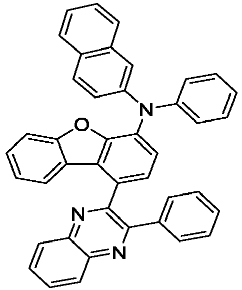 | 59% |
| 99 | 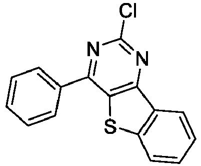 | 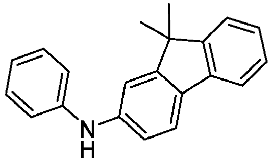 | 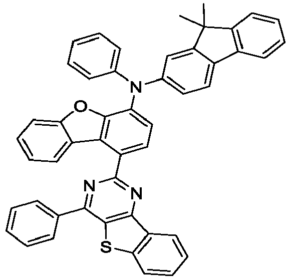 | 55% |
| 104 | 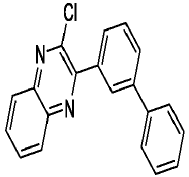 | 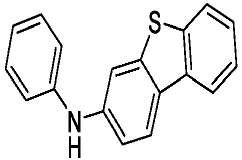 | 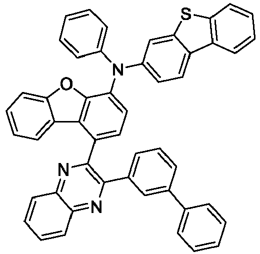 | 66% |
| 112 | 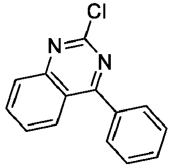 | 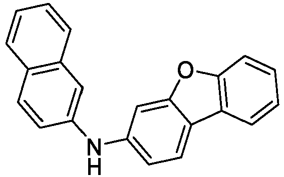 | 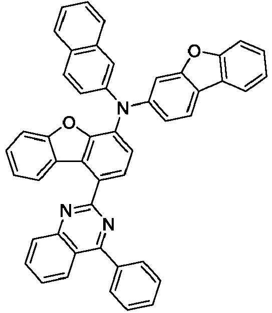 | 52% |
| 120 | 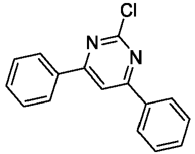 | 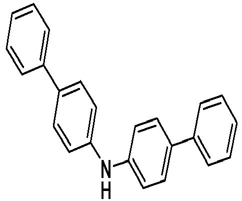 | 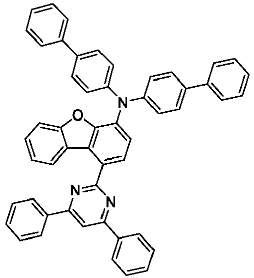 | 48% |
| 128 | 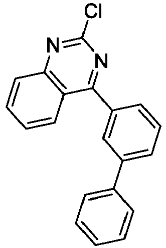 | 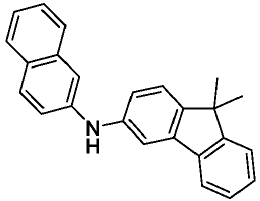 | 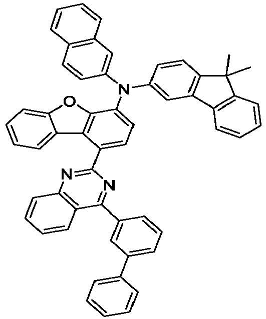 | 68% |
| 129 | 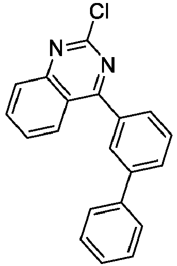 | 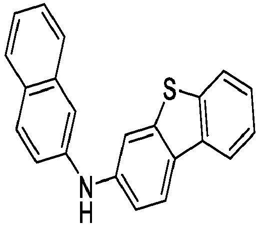 | 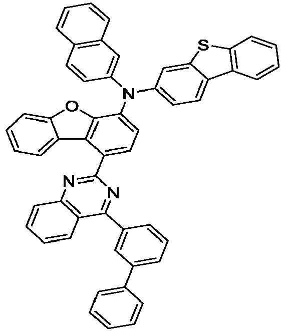 | 59% |
| 141 | 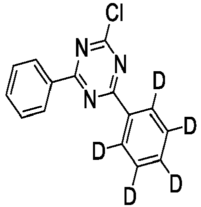 | 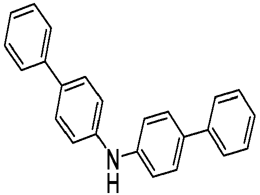 | 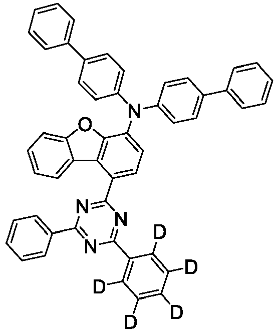 | 44% |
| 146 | 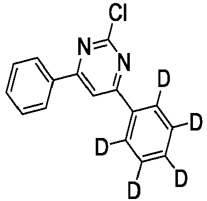 | 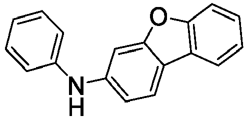 | 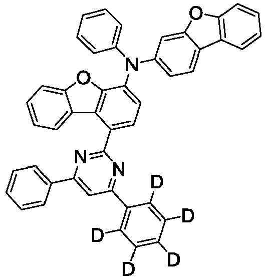 | 52% |
| 147 | 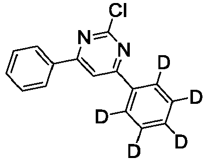 | 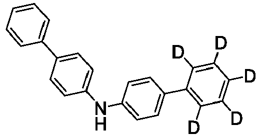 | 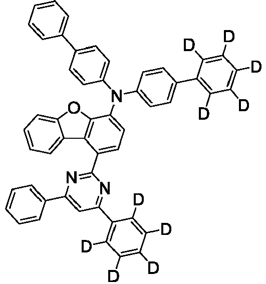 | 43% |

In addition, compounds not described in Preparation Examples 1 to 4 among the heterocyclic compounds represented by Chemical Formula 1 according to the present application were also prepared in the same manner as in the preparation examples.

In addition, the synthesis identification results are shown in the following Table 5 and Table 6. The following Table 5 shows measurement values of ¹H NMR (CDCl₃, 300 Mz), and the following Table 6 shows measurement values of FD-mass spectrometry (FD-MS: field desorption mass spectrometry).

**[Table 5]**

| NO | ¹H NMR (CDCl₃, 300 Mz) |
|---|---|
| 1 | 8.36 (d, 4H), 7.98 (d, 1H), 7.75 (d, 4H), 7.55-7.39 (m, 24H), 7.10 (d, 1H) |
| 4 | 8.36 (d, 2H), 7.98 (d, 2H), 7.78-7.75 (m, 4H), 7.55-7.24 (m, 20H), 7.10 (s, 1H), 7.08(d, 2H), 7.00 (dd, 1H) |
| 11 | 8.36 (d, 4H), 7.98 (d, 1H), 7.90-7.76 (m, 4H), 7.55-7.50 (m, 10H), 7.39-7.16 (m, 10H), 7.08-7.00 (m, 3H), 1.69 (s, 6H) |
| 12 | 8.36 (d, 4H), 7.98 (d, 1H), 7.90-7.86 (d, 2H), 7.59-7.50 (m, 9H), 7.39-7.10 (m, 12H), 1.69 (s, 6H) |
| 14 | 8.36 (d, 4H), 7.98 (d, 2H), 7.75 (d, 2H), 7.55-7.31 (m, 21H), 7.18 (t, 1H), 7.10 (d, 1H), 6.97 (dd, 1H) |
| 15 | 8.45 (d, 1H), 8.36 (m, 4H), 8.11 (dd, 1H), 7.98 (dd, 1H), 7.86 (dd, |
| | 1H), 7.75 (d, 2H), 7.56-7.50 (m, 10H), 7.49-7.41 (m, 4H), 7.39-7.27 (m, 7H), 7.10 (d, 1H) |
| 16 | 8.45 (d, 1H), 8.36 (m, 2H), 7.98-7.96 (m, 2H), 7.86-7.76 (m, 3H), 7.67 (d, 1H), 7.56-7.50 (m, 8H), 7.36-7.31 (m, 9H), 7.08-7.00 (m, 6H) |
| 20 | 8.36 (d, 4H), 7.98 (d, 1H), 7.82-7.75 (m, 4H), 7.55-7.50 (m, 11H), 7.49-7.41 (m, 3H), 7.39-7.31 (m, 2H), 7.27-7.24 (m, 6H), 7.08-7.00 (m, 3H) |
| 21 | 8.36 (d, 2H), 7.98 (d, 1H), 7.75 (d, 2H), 7.60-7.50 (m, 10H), 7.49-7.41 (m, 4H), 7.39-7.24 (m, 8H), 7.10-7.00 (m, 4H) |
| 27 | 8.97 (d, 1H), 8.36 (d, 2H), 8.00-7.94 (m, 3H), 7.84-7.75 (m, 6H), 7.55-7.27 (m, 23H), 7.10 (d, 1H), |
| 28 | 9.09 (s, 1H), 8.49 (d, 1H), 8.36 (d, 2H), 8.16 (d, 1H), 8.08 (d, 1H), 8.00-7.98 (m, 2H), 7.75 (d, 2H), 7.61-7.24 (m, 17H), 7.10-7.00 (m, 4H) |
| 32 | 8.45 (d, 1H), 8.36 (d, 4H), 8.11 (d, 1H), 7.98 (d, 1H), 7.86 (d, 1H), 7.59-7.50 (m, 9H), 7.41-.7.24 (m, 7H), 7.10-7.00 (m, 4H) |
| 33 | 8.36 (d, 4H), 7.98 (d, 2H), 7.64-7.50 (m, 10H), 7.39-7.08 (m, 12H), |
| 35 | 8.36 (d, 4H), 7.98 (d, 1H), 7.90-7.86 (m, 2H), 7.75 (d, 2H), 7.55-7.27 (m, 21H), 7.16 (d, 1H), 7.10 (d, 1H), 1.69 (s, 6H) |
| 39 | 8.45 (d, 1H), 8.36 (d, 4H), 7.98 (d, 1H), 7.86-7.74 (m, 4H), 7.64 (s, 1H), 7.54-7.24 (m, 18H), 7.24-7.08 (m, 4H) |
| 40 | 8.36 (d, 4H), 8.03 (s, 1H), 7.98 (s, 2H), 7.82 (d, 1H), 7.76 (d, 1H), 7.55-7.50 (m, 11H), 7.39-7.27 (m, 8H), 7.08-7.00 (m, 3H), 6.91 (d, 1H) |
| 47 | 8.55 (d, 1H), 8.36 (d, 2H), 8.19 (d, 1H), 7.98 (d, 1H), 7.82 (d, 1H), 7.76 (d, 1H), 7.55-7.50 (m, 7H), 7.40-7.00 (m, 19H), |
| 51 | 8.36 (d, 4H), 7.98 (d, 1H), 7.75 (d, 4H), 7.55-7.27 (m, 24H), 7.16 (d, 1H) |
| 52 | 8.36 (d, 4H), 7.98 (d, 1H), 7.82 (d, 1H), 7.76 (d, 1H), 7.75 (d, 4H), 7.55 (d, 4H), 7.54 (d, 1H), 7.50 (m, 4H), 7.49-7.41 (m, 6H), 7.39 (t, 1H), 7.31 (t, 1H), 7.27 (d, 6H) |
| 54 | 8.36 (d, 4H), 7.98 (d, 1H), 7.82 (d, 1H), 7.76 (d, 1H), 7.75 (d, 2H), 7.55 (d, 4H), 7.50 (m, 6H), 7.49-7.41 (m, 6H), 7.39 (t, 1H), 7.31 (t, 1H), 7.27 (d, 4H), 7.24 (t, 2H), 7.08-7.00 (m, 3H) |
| 55 | 8.36 (d, 4H), 7.98 (d, 1H), 7.82-7.75 (m, 4H), 7.55-7.17 (m, 22H), 7.08-7.00 (m, 3H) |
| 58 | 8.45 (d, 1H), 8.36 (d, 4H), 7.98 (d, 1H), 7.86-7.74 (m, 4H), 7.64 (s, 1H), 7.56-7.24 (m, 18H), 7.08-7.00 (m, 3H) |
| 60 | 8.36 (d, 2H), 8.08 (d, 1H), 7.98 (d, 2H), 7.75 (d, 4H), 7.63 (d, 1H), 7.55 (d, 4H), 7.54 (d, 2H), 7.49 (m, 4H), 7.41 (t, 4H), 7.39 (t, 2H), 7.31 (t, 2H), 7.16 (d, 1H) |
| 64 | 8.36 (d, 2H), 7.98 (d, 2H), 7.75 (d, 2H), 7.60 (d, 1H), 7.57 (d, 1H), 7.55 (d, 2H), 7.54 (d, 2H), 7.53 (d, 1H), 7.50 (t, 3H), 7.49 (t, 2H), 7.47 (t, 1H), 7.41 (t, 1H), 7.39 (t, 2H), 7.31 (t, 2H), 7.27 (d, 2H), 7.24 (t, 2H), 7.16 (d, 1H), 7.08-7.00 (m, 3H) |
| 67 | 8.45 (d, 1H), 8.36 (d, 2H), 7.98-7.96 (d, 2H), 7.86 (d, 1H), 7.75-7.27 (m, 26H), 7.16-7.11 (m, 2H) |
| 69 | 7.98 (d, 1H), 7.86-7.75 (d, 6H), 7.55-7.27 (m, 18H), 7.10-7.00 (m, 4H) |
| 74 | 8.38 (d, 1H), 8.03-7.98 (d, 3H), 7.80 (m, 3H), 7.71 (d, 1H), 7.67 (t, 2H), 7.62 (d, 1H), 7.59 (t, 1H), 7.55 (d, 2H), 7.54 (d, 2H), 7.45 (t, 1H), 7.42 (d, 1H), 7.39 (t, 1H), 7.38 (t, 1H), 7.32-7.31 (t, 3H), 7.27 (d, 2H), 7.24 (t, 2H), 7.11-7.00 (m, 4H) |
| 78 | 8.45 (d, 1H), 7.95 (d, 2H), 7.86-7.65 (m, 8H), 7.56-7.22 (m, 17H), 7.08-7.00 (m, 3H) |
| 80 | 8.35 (d, 2H), 8.23 (s, 1H), 7.94 (d, 2H), 7.75 (d, 2H), 7.55-7.27 (m, 24H), 7.10 (d, 2H) |
| 81 | 8.23 (s, 1H), 7.98 (d, 1H), 7.94 (d, 4H), 7.75 (d, 4H), 7.55-7.27 (m, 24H), 7.10 (d, 1H) |
| 82 | 8.23 (s, 1H), 7.98 (d, 1H), 7.94 (d, 4H), 7.75 (d, 2H), 7.55-7.27 (m, 19H), 7.10-7.00 (m, 4H) |
| 88 | 8.09-7.98 (m, 4H), 7.80-7.20 (m, 19H), 7.11-7.00 (m, 4H) |
| 89 | 8.32 (d, 1H), 8.03-7.98 (d, 3H), 7.86-7.80 (d, 3H), 7.67-7.24 (m, 20H), 7.11-7.00 (m, 4H) |
| 99 | 7.98 (d, 1H), 7.90-7.78 (d, 6H), 7.55-7.49 (m, 6H), 7.36-7.16 (m, 11H), 7.08-7.00 (m, 3H), 1.69 (s, 6H) |
| 104 | 8.50-8.45 (d, 3H), 8.09 (d, 1H), 7.98 (d, 1H), 7.86-7.20 (m, 23H), 7.08-7.00 (m, 3H) |
| 112 | 8.13 (d, 1H), 8.03 (s, 1H), 7.98 (d, 2H), 7.84-7.83 (t, 2H), 7.80 (d, 2H), 7.65-7.31 (m, 18H), 7.16-7.11 (d, 2H), 6.91 (d, 1H) |
| 113 | 8.35 (d, 2H), 7.98 (d, 1H), 7.90-7.65 (d, 6H), 7.55-7.54 (d, 3H), 7.50 (t, 3H), 7.38-7.22 (m, 12H), 7.08-7.00 (m, 3H), 1.69 (t, 6H) |
| 116 | 8.22 (d, 2H), 7.98-7.94 (d, 5H), 7.84-7.76 (d, 4H), 7.63-7.27 (m, 23H) |
| 120 | 8.23 (s, 1H), 7.98-7.94 (d, 5H), 7.75 (d, 4H), 7.55-7.27 (m, 24H), 7.16 (d, 1H) |
| 123 | 8.45 (d, 1H), 7.98-7.95 (d, 2H), 7.86-7.65 (d, 8H), 7.54-7.22 (m, |
| | 17H), 7.08-7.00 (m, 3H) |
| 126 | 8.45 (d, 1H), 8.13 (d, 1H), 7.98 (d, 1H), 7.95 (s, 1H), 7.89-7.80 (m, 8H), 7.65-7.24 (m, 16H), 7.08-7.00 (m, 3H) |
| 128 | 8.13 (d, 1H), 7.94-7.71 (m, 10H), 7.62-7.38 (m, 19H), 7.16-7.06 (m, 3H), 1.69 (t, 6H) |
| 129 | 8.45 (d, 1H), 8.13 (d, 1H), 7.94-7.31 (m, 29H), 7.16 (d, 1H), 7.11 (s, 1H) |
| 136 | 8.36 (d, 4H), 7.98 (d, 1H), 7.82-7.75 (d, 4H), 7.55-7.27 (m, 20H) |
| 139 | 8.36 (d, 2H), 7.98 (d, 1H), 7.82 (d, 1H), 7.76 (d, 1H), 7.55 (m, 2H), 7.39-7.24 (m, 5H), 7.08-7.00 (m, 3H) |
| 141 | 8.36 (d, 2H), 7.98 (d, 1H), 7.55-7.27 (m, 21H), 7.16 (d, 1H) |
| 146 | 8.23 (s, 1H), 8.03-7.94 (m, 5H), 7.55-7.49 (m, 7H), 7.39-7.16 (m, 7H), 7.08-7.00 (m, 3H), 6.91 (d, 1H) |
| 147 | 8.23 (s, 1H), 7.98-7.94 (m, 3H), 7.75 (d, 2H), 7.55-7.27 (m, 18H), 7.16 (d, 1H) |

**[Table 6]**

| Compo und | FD-MS | Compo und | FD-MS |
|---|---|---|---|
| 1 | m/z=718.27 (C₅₁H₃₄N₄O=718.86) | 2 | m/z=824.26 (C₅₇H₃₆N₄OS=825.00) |
| 3 | m/z=808.28 (C₅₇H₃₆N₄O₂=808.94) | 4 | m/z=732.25 (C₅₁H₃₂N₄O₂=732.84) |
| 5 | m/z=834.34 (C₆₀H₄₂N₄O=835.02) | 6 | m/z=882.34 (C₆₄H₄₂N₄O=883.07) |
| 7 | m/z=808.28 (C₅₇H₃₆N₄O₂=808.94) | 8 | m/z=748.23 (C₅₁H₃₂N₄OS=748.90) |
| 9 | m/z=794.30 (C₅₇H₃₈N₄O=794.96) | 10 | m/z=692.26 (C₄₉H₃₂N₄O=692.82) |
| 11 | m/z=758.30 (C₅₄H₃₈N₄O=758.93) | 12 | m/z=682.27 (C₄₈H₃₄N₄O=682.83) |
| 13 | m/z=666.24 (C₄₇H₃₈N₄O=666.78) | 14 | m/z=732.25 (C₅₁H₃₂N₄O₂=732.84) |
| 15 | m/z=748.23 (C₅₁H₃₂N₄OS=748.90) | 16 | m/z=748.23 (C₅₁H₃₂N₄OS=748.90) |
| 17 | m/z=731. 27 (C₅₁H₃₃N₅O=731.86) | 18 | m/z=781.28 (C₅₅H₃₅N₅O=781.92) |
| 19 | m/z=857.32 (C₆₁H₃₉N₅O=858.02) | 20 | m/z=718.27 (C₅₁H₃₄N₄O=718.86) |
| 21 | m/z=732.25 (C₅₁H₃₂N₄O₂=732.84) | 22 | m/z=824.26 (C₅₇H₃₆N₄OS=825.00) |
| 23 | m/z=794.30 (C₅₇H₃₈N₄O=794.96) | 24 | m/z=794.30 (C₅₇H₃₈N₄O=794.96) |
| 25 | m/z=794.30 (C₅₇H₃₈N₄O=794.96) | 26 | m/z=794.30 (C₅₇H₃₈N₄O=794.96) |
| 27 | m/z=768.29 (C₅₅H₃₆N₄O=768.92) | 28 | m/z=692.26 (C₄₉H₃₂N₄O=692.82) |
| 29 | m/z=719.27 (C₅₀H₃₃N₅O=719.85) | 30 | m/z=870.34 (C₆₃H₄₂N₄O=871.06) |
| 31 | m/z=566.21 (C₃₉H₂₆N₄O=566.66) | 32 | m/z=672.20 (C₄₅H₂₈N₄OS=672.81) |
| 33 | m/z=656.22 (C₄₅H₂₈N₄O₂=656.75) | 34 | m/z=762.21 (C₅₄H₃₀N₄O₂S=762.89) |
| 35 | m/z=758.30 (C₅₄H₃₈N₄O=758.93) | 36 | m/z=794.30 (C₅₇H₃₈N₄O=794.96) |
| 37 | m/z=794.30 (C₅₇H₃₈N₄O=794.96) | 38 | m/z=642.24 (C₄₅H₃₀N₄O=642.76) |
| 39 | m/z=748.23 (C₅₁H₃₂N₄OS=748.90) | 40 | m/z=732.25 (C₅₁H₃₂N₄O₂=732.84) |
| 41 | m/z=768.29 (C₅₅H₃₆N₄O=768.92) | 42 | m/z=718.27 (C₅₁H₃₄N₄O=718.86) |
| 43 | m/z=794.30 (C₅₇H₃₈N₄O=794.96) | 44 | m/z=794.30 (C₅₇H₃₈N₄O=794.96) |
| 45 | m/z=795.30 (C₅₆H₃₇N₅O=795.95) | 46 | m/z=742.27 (C₅₃H₃₄N₄O=742.88) |
| 47 | m/z=731. 27 (C₅₁H₃₃N₅O=731.86) | 48 | m/z=807.30 (C₅₇H₃₇N₅O=807.96) |
| 49 | m/z=768.29 (C₅₅H₃₆N₄O=768.92) | 50 | m/z=742.27 (C₅₃H₃₄N₄O=742.88) |
| 51 | m/z=718.27 (C₅₁H₃₄N₄O=718.86) | 52 | m/z=794.30 (C₅₇H₃₈N₄O=794.96) |
| 53 | m/z=794.30 (C₅₇H₃₈N₄O=794.96) | 54 | m/z=718.27 (C₅₁H₃₄N₄O=718.86) |
| 55 | m/z=718.27 (C₅₁H₃₄N₄O=718.86) | 56 | m/z=758.30 (C₅₄H₃₈N₄O=758.93) |
| 57 | m/z=645.25 (C₄₇H₃₅NS=645.85) | 58 | m/z=719.26 (C₅₃H₃₇NS=719.93) |
| 59 | m/z=692.26 (C₄₉H₃₂N₄O=692.82) | 60 | m/z=808.28 (C₅₇H₃₆N₄O₂=808.94) |
| 61 | m/z=782.27 (C₅₅H₃₄N₄O2=782.90) | 62 | m/z=808.28 (C₅₇H₃₆N₄O₂=808.94) |
| 63 | m/z=782.27 (C₅₅H₃₄N₄O2=782.90) | 64 | m/z=782.27 (C₅₅H₃₄N₄O₂=782.90) |
| 65 | m/z=748.23 (C₅₁H₃₂N₄OS=748.90) | 66 | m/z=798.25 (C₅₅H₃₄N₄OS=798.96) |
| 67 | m/z=798.25 (C₅₅H₃₄N₄OS=798.96) | 68 | m/z=748.23 (C₅₁H₃₂N₄OS=748.90) |
| 69 | m/z=671.20 (C₄₆H₂₉N₃OS=671.82) | 70 | m/z=639.23 (C₄₆H₂₉N₃O=639.76) |
| 71 | m/z=747.23 (C₅₆H₄₁NOS=747.92) | 72 | m/z=747.23 (C₅₂H₃₃N₃OS=747.92) |
| 73 | m/z=767.29 (C₅₆H₃₇N₃O=767.93) | 74 | m/z=665.25 (C₄₈H₃₁N₃O=665.80) |
| 75 | m/z=741.28 (C₅₄H₃₅N₃O=741.89) | 76 | m/z=691.26 (C₅₀H₃₃N₃O=691.83) |
| 77 | m/z=665.25 (C₄₈H₃₄N₃O=665.80) | 78 | m/z=761. 21 (C₅₂H₃₁N₃O₂S=761.90) |
| 79 | m/z=691.26 (C₅₀H₃₃N₃O=691.83) | 80 | m/z=717.28 (C₅₂H₃₅N₃O=717.87) |
| 81 | m/z=717.28 (C₅₂H₃₅N₃O=717.87) | 82 | m/z=641.25 (C₄₆H₃₁N₃O=695.91) |
| 83 | m/z=615.23 (C₄₄H₂₉N₃O=615.74) | 84 | m/z=620.19 (C₄₃H₂₈N₂OS=620.77) |
| 85 | m/z=615.23 (C₄₄H₂₉N₃O=615.74) | 86 | m/z=665.25 (C₄₈H₃₁N₃O=665.80) |
| 87 | m/z=755.29 (C₅₅H₃₇N₃O=755.92) | 88 | m/z=589.22 (C₄₂H₂₇N₃O=589.70) |
| 89 | m/z=665.25 (C₄₈H₃₁N₃O=665.80) | 90 | m/z=741.28 (C₅₄H₃₅N₃O=741.89) |
| 91 | m/z=615.23 (C₄₄H₂₉N₃O=615.74) | 92 | m/z=691.26 (C₅₀H₃₃N₃O=691.83) |
| 93 | m/z=665.25 (C₄₈H₃₁N₃O=665.80) | 94 | m/z=695.26 (C₄₉H₃₃N₃O₂=695.82) |
| 95 | m/z=665.25 (C₄₈H₃₁N₃O=665.80) | 96 | m/z=665.25 (C₄₈H₃₁N₃O=665.80) |
| 97 | m/z=781.27 (C₅₆H₃₅N₃O₂=781.92) | 98 | m/z=721.22 (C₅₀H₃₁N₃OS=721.88) |
| 99 | m/z=711.23 (C₄₉H₃₃N₃OS=711.88) | 100 | m/z=787.27 (C₅₅H₃₇N₃OS=787.98) |
| 101 | m/z=665.25 (C₄₈H₃₁N₃O=665.80) | 102 | m/z=655.23 (C₄₈H₂₉N₃O₂=655.76) |
| 103 | m/z=731.29 (C₅₃H₃₇N₃O=731.90) | 104 | m/z=721.22 (C₅₀H₃₁N₃OS=721.88) |
| 105 | m/z=655.26 (C₄₇H₃₃N₃O=655.80) | 106 | m/z=715.26 (C₅₂H₃₃N₃O=715.86) |
| 107 | m/z=731. 26 (C₅₂H₃₃N₃O₂=731.86) | 108 | m/z=747.23 (C₅₂H₃₃N₃OS=747.92) |
| 109 | m/z=690.27 (C₅₁H₃₄N₂O=690.85) | 110 | m/z=741.28 (C₅₄H₃₅N₃O=741.89) |
| 111 | m/z=705.24 (C₅₈H₃₁N₃O₂=705.82) | 112 | m/z=679.23 (C₄₈H₂₉N₃O₂=679.78) |
| 113 | m/z=771.29 (C₅₅H₃₇N₃O₂=771.92) | 114 | m/z=781.31 (C₅₇H₃₉N₃O=781.96) |
| 115 | m/z=705.24 (C₅₀H₃₁N₃O₂=705.82) | 116 | m/z=741.28 (C₅₄H₃₅N₃O=741.89) |
| 117 | m/z=757.31 (C₅₅H₃₉N₃O=757.94) | 118 | m/z=747.23 (C₅₂H₃₃N₃OS=747.92) |
| 119 | m/z=655.23 (C₄₆H₂₉N₃O₂=655.76) | 120 | m/z=717.28 (C₅₂H₃₅N₃O=717.87) |
| 121 | m/z=721.22 (C₅₀H₃₁N₃OS=721.88) | 122 | m/z=695.20 (C₄₈H₂₉N₃OS=695.84) |
| 123 | m/z=761. 21 (C₅₂H₃₁N₃O₂S=761.90) | 124 | m/z=685.18 (C₄₆H₂₇N₃O₂S=685.80) |
| 125 | m/z=761. 21 (C₅₂H₃₁N₃O₂S=761.90) | 126 | m/z=721.22 (C₅₀H₃₁N₃OS=721.88) |
| 127 | m/z=679.26 (C₄₉H₃₃N₃O=679.82) | 128 | m/z=781.31 (C₅₇H₃₉N₃O=781.96) |
| 129 | m/z=771.23 (C₅₄H₃₃N₃OS=771.94) | 130 | m/z=721.22 (C₅₀H₃₁N₃OS=721.88) |
| 131 | m/z=691.26 (C₅₀H₃₃N₃O=691.83) | 132 | m/z=747.23 (C₅₂H₃₃N₃OS=747.92) |
| 133 | m/z=747.23 (C₅₂H₃₃N₃OS=747.92) | 134 | m/z=723.30 (C₅₁H₂₉D₅N₄O=723.89) |
| 135 | m/z=697.29 (C₄₉H₂₇D₅N₄O =697.85) | 136 | m/z=723.30 (C₅₁H₂₉D₅N₄O =723.89) |
| 137 | m/z=799.34 (C₅₇H₃₃D₅N₄O=799.99) | 138 | m/z=767.24 (C₅₁H₂₅D₅N₄O₂S =767.92) |
| 139 | m/z=652.30 (C₄₅H₂₀D₁₀N₄O =652.82) | 140 | m/z=799.34 (C₅₇H₃₃D₅N₄O=799.99) |
| 141 | m/z=723.30 (C₅ᵢH₂₉D₅N₄O =723.89) | 142 | m/z=813.32 (C₅₇H₃₁D₅N₄O₂ =813.97) |
| 143 | m/z=670.28 (C₄₈H₂₆D₅N₃O =670. 83) | 144 | m/z=726.25 (C₅₀H₂₆D₅N₃OS =726.91) |
| 145 | m/z=684.26 (C₄₈H₂₄D₅N₃O₂ =684.81) | 146 | m/z=660.26 (C₄₆H₂₄D₅N₃O₂ =660.79) |
| 147 | m/z=727.34 (C₅₂H₂₅D₁₀N₃O =727.93) | | |

### <Preparation Example 5> Preparation of Target Compound 1-1

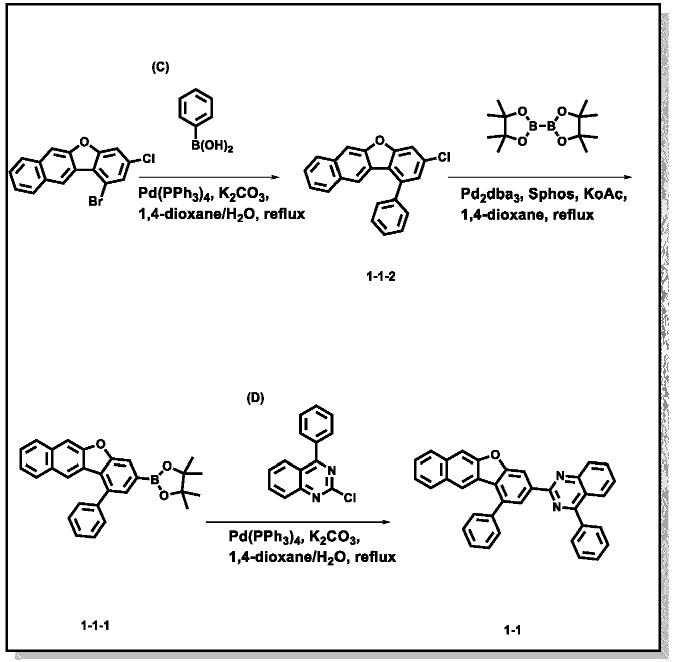

### 1) Preparation of Compound 1-1-2

1-Bromo-3-chloronaphtho[2,3-b]benzofuran (6.0 g, 18.09 mmol), phenylboronic acid (C) (2.65 g, 21.71 mmol), Pd(PPh₃)₄ (1.23 g, 1.07 mmol) and K₂CO₃ (5.89 g, 42.62 mmol) were dissolved in 1,4-dioxane/H₂O (30 mL/6 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and the reaction material was purified once again by column chromatography (DCM:Hex=1:5) to obtain Compound 1-1-2 (5 g, 45%). The Hex means hexane.

### 2) Preparation of Compound 1-1-1

Compound 1-1-2 (5.0 g, 15.21 mmol), bis(pinacolato)diboron (4.3 g, 16.73 mmol), Pd₂(dba)₃ (0.44 g, 1.07 mmol), Sphos (0.4 g, 0.958 mmol) and KOAc (1.9 g, 19.16 mmol) were dissolved in 1,4-dioxane (50 mL), and refluxed for 5 hours. After the reaction was completed, the result was extracted by introducing distilled water and dichloromethane (DCM) thereto at room temperature, and after drying the organic layer with MgSO₄, the solvent was removed using a rotary evaporator. The solvent-removed reaction material was silica purified, and then recrystallized with methanol to obtain Compound 1-1-1 (3.27 g, 56%).

### 3) Preparation of Target Compound 1-1

Compound 1-1-1 (3.27 g, 7.8 mmol), 2-chloro-4-phenylquinazoline (D) (1.9 g, 7.8 mmol), Pd(PPh₃)₄ (0.31 g, 0.27 mmol) and K₂CO₃ (1.47 g, 10.66 mmol) were dissolved in 1,4-dioxane/H₂O (25 mL/5 mL), and refluxed for 3 hours. After the reaction was completed, produced solids were filtered, washed with distilled water, and dried. The dried solids were dissolved in chloroform for silica purification, and the solvent was removed using a rotary evaporator. The result was recrystallized with acetone to obtain target Compound 1-1 (3.1 g, 80%).

### 4) Preparation of Additional Target Compounds

Target compounds of the following Table 7 were additionally synthesized in the same manner as in Preparation Example 5 except that C and D of the following Table 7 were used as intermediates instead of using phenylboronic acid (C) and 2-chloro-4-phenylquinazoline (D) as Intermediates C and D.

**[Table 7]**

| Compo und No. | Intermediate C | Intermediate D | Target Compound | Yield |
|---|---|---|---|---|
| 1-2 | 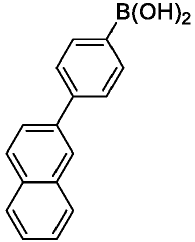 | 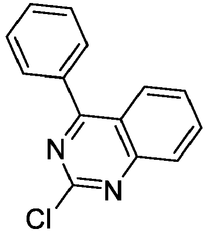 | 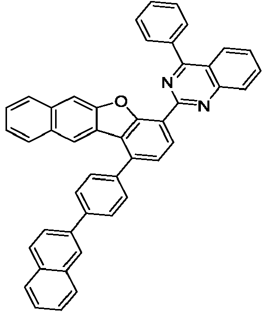 | 63% |
| 1-3 | 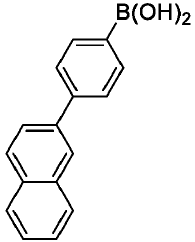 | 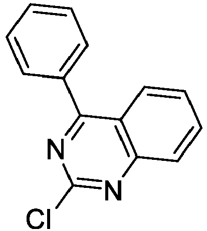 | 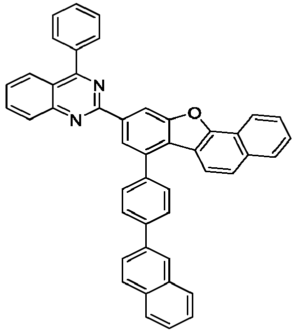 | 59% |
| 1-5 | 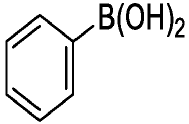 | 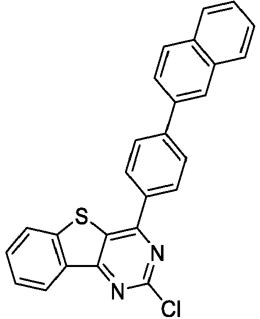 | 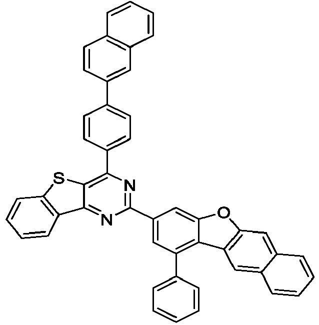 | 69% |
| 1-6 | 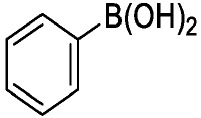 | 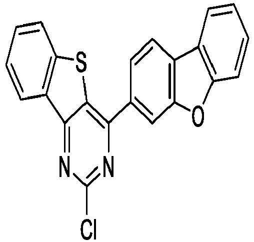 | 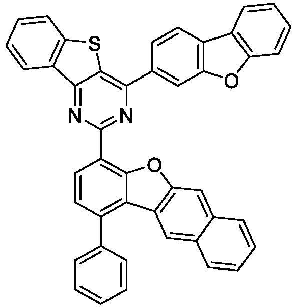 | 48% |

In addition, compounds not described in Preparation Example 5 among the heterocyclic compounds represented by Chemical Formulae 4 to 6 according to the present application were also prepared in the same manner as in the preparation examples.

In addition, the synthesis identification results are shown in the following Table 8 and Table 9. The following Table 8 shows measurement values of ¹H NMR (CDCl₃, 300 Mz), and the following Table 9 shows measurement values of FD-mass spectrometry (FD-MS: field desorption mass spectrometry).

**[Table 8]**

| NO | ¹H NMR (CDCl₃, 300 Mz) |
|---|---|
| 1-1 | 8.28 (d, 1H), 8.13 (d, 1H), 8.86-7.75 (m, 10H), 7.55-7.39 (m, 24H), 7.65-7.41 (m, 10H) |
| 1-5 | 8.30 (d, 2H), 8.09 (d, 1H), 8.06 (d, 1H), 7.99 (d, 1H), 7.86-7.75 (m, 10H), 7.63-7.31(m, 12H), |
| 1-6 | 8.28 (d, 1H), 7.98 (d, 1H), 7.86-7.75 (m, 10H), 7.54-7.31 (m, 12H) |
| 1-7 | 8.97 (d, 2H), 8.95 (d, 1H), 8.50 (dd, 1H), 7.86-7.76 (m, 9H), 7.53-7.25 (m, 15H) |
| 1-8 | 8.95 (dd, 1H), 8.50 (dd, 1H), 8.36 (d, 2H), 8.25 (d, 2H), 8.19 (d, 1H), 7.89-7.75 (m, 8H), 7.50-7.35 (m, 10H), 7.25 (d, 2H), 7.23 (d, 1H), |
| 1-9 | 8.13 (d, 1H), 8.04-7.94 (m, 6H), 7.84-7.75 (m, 7H), 7.59-7.31 (m, 12H) |
| 1-10 | 8.95 (d, 2H), 8.86 (d, 4H), 8.50 (d, 2H), 7.89-7.67 (m, 10H), 7.41-7.29 (m, 8H), |
| 1-13 | 8.38 (d, 1H), 8.08-7.71 (d, 13H), 7.61-7.54 (d, 4H), 7.79-7.31 (m, 8H) |
| 1-18 | 8.93 (d, 1H), 8.36 (d, 4H), 8.25 (d, 2H), 8.03-7.95 (d, 3H), 7.54-7.31 (m, 17H) |
| 1-19 | 9.27 (d, 1H), 8.79 (d, 1H), 8.33-8.25 (m, 7H), 8.15 (d, 1H), 7.75-7.64 (m, 8H), 7.52-7.41 (m, 7H), 7.25 (d, 4H), |

**[Table 9]**

| Compo und | FD-MS | Compo und | FD-MS |
|---|---|---|---|
| 1-1 | m/z=498.17 (C₃₆H₂₂N₂O=498.59) | 1-5 | m/z=680.19 (C₄₈H₂₈N₂OS=680.83) |
| 1-6 | m/z=644.16 (C₄₄H₂₄N₂O₂S=644.75) | 1-7 | m/z=680.19 (C₄₈H₂₈N₂OS=680.83) |
| 1-8 | m/z=651.23 (C₄₇H₂₉N₃O=651.77) | 1-9 | m/z=574.20 (C₄₂H₂₈N₂O=574.68) |
| 1-10 | m/z=534.21 (C₄₀H₂₆N₂=534.66) | 1-13 | m/z=670.17 (C₄₆H₂₆N₂O₂S=670.79) |
| 1-18 | m/z=601.22 (C₄₃H₂₇N₃O=601.71) | 1-19 | m/z=611.24 (C₄₅H₂₉N₃=611.75) |

### <Experimental Example 1>

### (1) Manufacture of Organic Light Emitting Device (Red Host)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. In other words, the light emitting layer was thermal vacuum deposited to a thickness of 500 Å using compounds described in the following Table 10 and Table 11 as a host, and doping (piq)₂(Ir) (acac) as a red phosphorescent dopant to the host by 3 wt% based on a total weight of the light emitting layer.

After that, BCP (bathocuproine, 2,9-dimethyl-4,7-diphenyl-1,10-phenanthroline) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured (Comparative Examples 1 to 9 and Examples 1 to 43).

Meanwhile, all the organic compounds required to manufacture the OLED were vacuum sublimation purified under 10⁻⁸ torr to 10⁻⁶ torr for each material to be used in the OLED manufacture.

Herein, the following Table 10 corresponds to cases of using a single host material, and Table 11 corresponds to cases of using the compound corresponding to Chemical Formula 1 (donor (p-host)) of the present application having a favorable hole transfer ability as a first host and the compound corresponding to any one of Chemical Formulae 4 to 6 (acceptor (n-host)) of the present application having a favorable electron transfer ability as a second host, and depositing the two host compounds as one source of supply.

Herein, Comparative Compounds A to I used in Comparative Examples 1 to 9 are as follows.

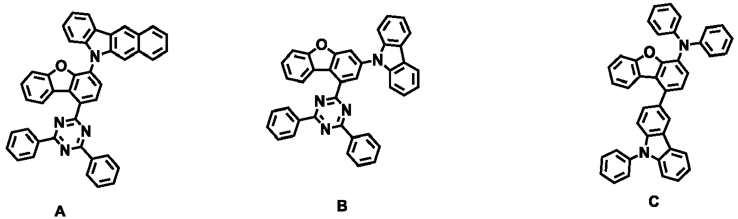

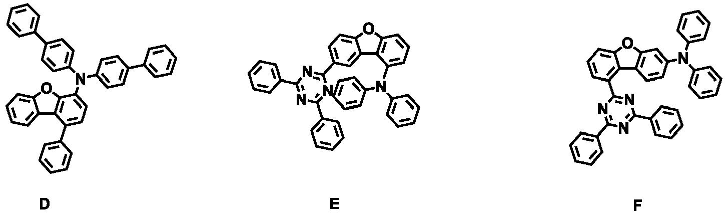

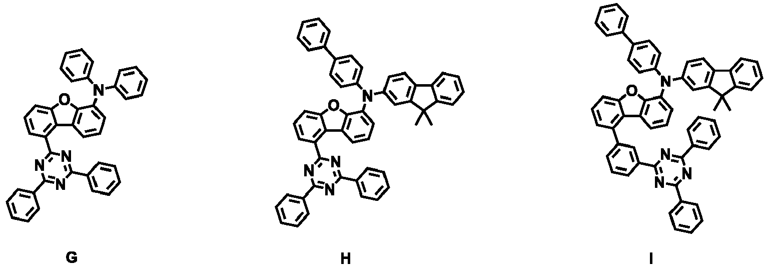

### (2) Driving Voltage and Light Emission Efficiency of Organic Electroluminescent Device

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. Herein, T90 means a lifetime (unit: h, time), a time taken to become 90% with respect to initial luminance.

Properties of the organic light emitting devices of the present disclosure obtained as a result of the measurements are as shown in the following Table 10 and Table 11.

**[Table 10]**

| | Compound | Driving Voltage (V) | Efficie ncy (cd/A) | Color Coordinate (x, y) | Lifetime (T90) |
|---|---|---|---|---|---|
| Comparative Example 1 | A | 4.39 | 12.6 | (0.682, 0.317) | 58 |
| Comparative Example 2 | B | 4.36 | 9.7 | (0.691, 0.309) | 51 |
| Comparative Example 3 | C | 4.08 | 6.8 | (0.675, 0.325) | 21 |
| Comparative Example 4 | D | 4.01 | 5.5 | (0.681, 0.319) | 32 |
| Comparative Example 5 | E | 3.98 | 11.2 | (0.675, 0.325) | 35 |
| Comparative Example 6 | F | 4.03 | 9.5 | (0.684, 0.316) | 39 |
| Comparative Example 7 | G | 4.04 | 12.3 | (0.691, 0.309) | 33 |
| Comparative Example 8 | H | 3.93 | 9.6 | (0.683, 0.317) | 25 |
| Comparative Example 9 | I | 4.05 | 10.5 | (0.681, 0.319) | 13 |
| Example 1 | 1 | 3.75 | 19.5 | (0.682, 0.317) | 102 |
| Example 2 | 4 | 3.84 | 24.7 | (0.675, 0.325) | 98 |
| Example 3 | 11 | 3.91 | 28.7 | (0.682, 0.317) | 86 |
| Example 4 | 12 | 3.85 | 20.1 | (0.691, 0.309) | 85 |
| Example 5 | 14 | 4.03 | 23.2 | (0.691, 0.309) | 102 |
| Example 6 | 15 | 4.05 | 22.8 | (0.675, 0.325) | 114 |
| Example 7 | 16 | 3.82 | 23.8 | (0.681, 0.319) | 111 |
| Example 8 | 20 | 3.96 | 26.5 | (0.682, 0.317) | 99 |
| Example 9 | 21 | 4.07 | 21.5 | (0.675, 0.325) | 105 |
| Example 10 | 27 | 4.09 | 20.9 | (0.669, 0.321) | 108 |
| Example 11 | 28 | 4.13 | 20.1 | (0.681, 0.319) | 110 |
| Example 12 | 31 | 3.78 | 20.4 | (0.691, 0.309) | 100 |
| Example 13 | 33 | 4.07 | 21.5 | (0.681, 0.319) | 96 |
| Example 14 | 35 | 4.00 | 20.5 | (0.678, 0.322) | 95 |
| Example 15 | 39 | 4.01 | 24.8 | (0.674, 0.326) | 98 |
| Example 16 | 40 | 3.98 | 23.5 | (0.682, 0.317) | 101 |
| Example 17 | 47 | 4.04 | 23.9 | (0.691, 0.309) | 92 |
| Example 18 | 51 | 4.10 | 21.6 | (0.675, 0.325) | 90 |
| Example 19 | 52 | 4.02 | 26.5 | (0.681, 0.319) | 96 |
| Example 20 | 54 | 4.08 | 22.9 | (0.675, 0.325) | 95 |
| Example 21 | 55 | 4.12 | 22.5 | (0.669, 0.321) | 88 |
| Example 22 | 58 | 4.02 | 25.1 | (0.691, 0.309) | 89 |
| Example 23 | 60 | 4.11 | 20.5 | (0.681, 0.319) | 89 |
| Example 24 | 64 | 4.00 | 20.4 | (0.678, 0.322) | 97 |
| Example 25 | 67 | 4.18 | 20.5 | (0.685, 0.315) | 91 |
| Example 26 | 69 | 3.81 | 22.4 | (0.668, 0.351) | 100 |
| Example 27 | 74 | 4.09 | 24.5 | (0.685, 0.315) | 101 |
| Example 28 | 78 | 3.97 | 23.2 | (0.691, 0.309) | 93 |
| Example 29 | 80 | 3.91 | 21.8 | (0.681, 0.319) | 99 |
| Example 30 | 81 | 3.86 | 19.9 | (0.678, 0.322) | 102 |
| Example 31 | 82 | 3.92 | 20.5 | (0.674, 0.326) | 107 |
| Example 32 | 88 | 4.09 | 21.9 | (0.683, 0.317) | 103 |
| Example 33 | 89 | 3.95 | 24.6 | (0.681, 0.319) | 113 |
| Example 34 | 99 | 3.98 | 22.1 | (0.685, 0.315) | 87 |
| Example 35 | 104 | 4.06 | 21.2 | (0.676, 0.324) | 106 |
| Example 36 | 112 | 3.82 | 20.4 | (0.674, 0.326) | 93 |
| Example 37 | 113 | 4.08 | 23.2 | (0.691, 0.309) | 88 |
| Example 38 | 116 | 4.22 | 24.8 | (0.681, 0.319) | 106 |
| Example 39 | 120 | 4.15 | 21.5 | (0.685, 0.315) | 97 |
| Example 40 | 123 | 4.08 | 24.6 | (0.691, 0.309) | 104 |
| Example 41 | 124 | 3.96 | 21.0 | (0.674, 0.326) | 94 |
| Example 42 | 128 | 4.01 | 21.5 | (0.683, 0.317) | 99 |
| Example 43 | 129 | 3.92 | 22.1 | (0.669, 0.321) | 96 |

**[Table 11]**

| | First Host | Second Host | Ratio (N:P) | Driving Voltage (V) | Efficiency (cd/A) | Color Coordinate (x, y) | Lifetime (T₉₅) |
|---|---|---|---|---|---|---|---|
| Comparative Example 10 | A | 1-1 | 1:1 | 4.31 | 12.9 | (0.669, 0.321) | 70 |
| Comparative Example 11 | E | | | 3.92 | 15.5 | (0.691, 0.309) | 55 |
| Comparative Example 12 | H | | | 3.89 | 12.3 | (0.681, 0.319) | 40 |
| Example 44 | 31 | | | 3.70 | 26.9 | (0.678, 0.322) | 180 |
| Example 45 | 20 | 1-3 | 1:2 | 3.84 | 35.8 | (0.685, 0.315) | 190 |
| Example 46 | | 1-5 | | 3.79 | 38.8 | (0.668, 0.351) | 220 |
| Example 47 | | 1-8 | | 3.81 | 38.0 | (0.681, 0.319) | 216 |
| Example 48 | 26 | 1-13 | 1:3 | 4.01 | 22.4 | (0.683, 0.317) | 211 |
| Example 49 | 30 | 1-20 | | 4.06 | 21.1 | (0.675, 0.325) | 196 |
| Example 50 | 35 | 1-15 | | 3.94 | 22.9 | (0.684, 0.316) | 202 |
| Example 51 | 47 | 1-7 | | 3.99 | 25.5 | (0.682, 0.317) | 208 |
| Example 52 | 51 | 1-6 | 1:2 | 3.98 | 30.3 | (0.675, 0.325) | 224 |
| Example 53 | 52 | | | 3.86 | 36.6 | (0.682, 0.317) | 250 |
| Example 54 | 60 | 1-14 | 1:1 | 4.02 | 24.5 | (0.691, 0.309) | 190 |
| Example 55 | 62 | | | 4.03 | 23.4 | (0.681, 0.319) | 196 |
| Example 56 | 64 | | | 3.96 | 25.4 | (0.682, 0.317) | 208 |
| Example 57 | 67 | | | 4.08 | 23.0 | (0.691, 0.309) | 210 |
| Example 58 | 74 | 1-17 | 2:1 | 4.01 | 25.8 | (0.684, 0.316) | 214 |
| Example 59 | 75 | 1-4 | 2:1 | 4.08 | 24.5 | (0.683, 0.317) | 185 |
| Example 60 | 79 | 1-19 | 1:3 | 3.86 | 22.4 | (0.669, 0.321) | 206 |
| Example 61 | 80 | 1-20 | 1:3 | 3.88 | 23.1 | (0.675, 0.325) | 211 |
| Example 62 | 88 | 1-8 | 1:1 | 4.01 | 22.6 | (0.681, 0.319) | 220 |
| Example 63 | 89 | 1-5 | 1:2 | 3.88 | 25.6 | (0.684, 0.316) | 236 |
| Example 64 | 96 | 1-18 | 1:3 | 4.03 | 21.9 | (0.669, 0.321) | 208 |
| Example 65 | 104 | 1-16 | 1:3 | 3.99 | 24.1 | (0.684, 0.316) | 225 |
| Example 66 | 112 | 1-10 | 1:1 | 3.76 | 22.4 | (0.682, 0.317) | 149 |
| Example 67 | 116 | 1-9 | 1:1 | 4.11 | 25.9 | (0.691, 0.309) | 188 |
| Example 68 | 119 | 1-2 | 1:2 | 3.93 | 21.8 | (0.675, 0.325) | 169 |
| Example 69 | 123 | 1-12 | 1:2 | 3.98 | 26.8 | (0.681, 0.319) | 239 |
| Example 70 | 130 | 1-11 | 1:2 | 3.90 | 22.5 | (0.675, 0.325) | 156 |

In addition, HOMO (Highest Occupied Molecular Orbital), LUMO (Lowest Unoccupied Molecular Orbital) and band gap of each of the heterocyclic compounds of the present disclosure and the comparative example compounds are as shown in the following Table 12.

**[Table 12]**

| Compound | HOMO (eV) | LUMO (eV) | Band Gap | T1 (eV) |
|---|---|---|---|---|
| Comparative Compound A | -5.39 | -2.59 | 2.80 | 2.29 |
| Comparative Compound B | -5.33 | -2.07 | 3.26 | 2.65 |
| Comparative Compound C | -5.27 | -1.91 | 3.36 | 2.62 |
| Comparative Compound D | -5.12 | -1.92 | 3.48 | 2.72 |
| Comparative Compound E | -5.29 | -1.76 | 3.27 | 2.55 |
| Comparative Compound F | -5.31 | -2.05 | 3.33 | 2.53 |
| Comparative Compound G | -5.27 | -2.01 | 3.34 | 2.49 |
| Comparative Compound H | -5.29 | -2.11 | 3.28 | 2.52 |
| Comparative Compound I | -5.32 | -2.22 | 3.14 | 2.47 |
| Compound 20 of Present Application | -5.16 | -2.35 | 2.81 | 2.33 |
| Compound 51 of Present Application | -5.26 | -2.58 | 2.68 | 2.30 |
| Compound 52 of Present Application | -5.13 | -2.43 | 2.70 | 2.27 |

As seen from Table 12, it is identified that the heterocyclic compound of the present disclosure has a high HOMO level when, as a donor, having arylamine with a more favorable hole transfer ability, that is, higher donating strength as a substituent since a balance effect with an acceptor increases.

Accordingly, as seen from Table 10, the device using the heterocyclic compound of the present disclosure in the organic material layer has more reduced driving voltage and increased lifetime compared to the device using Comparative Compounds A and B having carbazole, a donor with weaker donating strength, as a substituent in the organic material layer, and the heterocyclic compound of the present disclosure is suitable as a red host of an organic light emitting device.

In addition, when compared with the compound of the present application, Compounds C and D of the comparative examples do not have an acceptor having a favorable electron transfer ability, and therefore, electron injection does not occur, and as seen from Table 10, efficiency and lifetime of the device using Comparative Compounds C and D in the organic material layer decrease.

In addition, it is identified that, although Compounds E to I of the comparative examples has a decreased driving voltage as seen from Table 10, efficiency and lifetime are low despite the substitution by arylamine having strong donor properties. This is due to the fact that, the T1 level is high depending on the substituent position of the heterocyclic compound core making energy transfer to the red dopant difficult, and a large band gap leads to high resistance adversely affecting the lifetime due to decreased stability.

Accordingly, it is identified that the compound of the present application has more reduced band gap and smaller T1 level, which significantly improves lifetime and efficiency.

In addition, as seen from Table 11, it is identified that comprising the heterocyclic compound of Chemical Formula 1 and the heterocyclic compound corresponding to any one of Chemical Formulae 4 to 6 at the same time in the organic material layer of the organic light emitting device improves driving voltage, efficiency and lifetime.

Such results may lead to a forecast that an exciplex phenomenon occurs by comprising the two compounds at the same time. The exciplex phenomenon is a phenomenon of releasing energy having sizes of a donor (p-host) HOMO level and an acceptor (n-host) LUMO level due to electron exchanges between a molecule having strong donor properties and a molecule having strong acceptor properties. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

Particularly, it was identified that the heterocyclic compound corresponding to any one of Chemical Formulae 4 to 6 was more effective in improving a lifetime when triazine or benzothieno pyrimidine, which is an n-host and strong acceptor, was present compared to when quinazoline was present.

When the exciplex phenomenon occurs between two molecules as above, reverse intersystem crossing (RISC) occurs, and as a result, internal quantum efficiency may increase up to 100%.

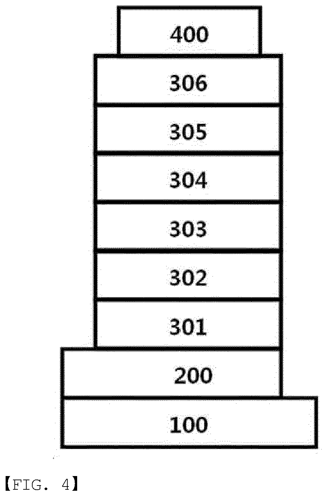

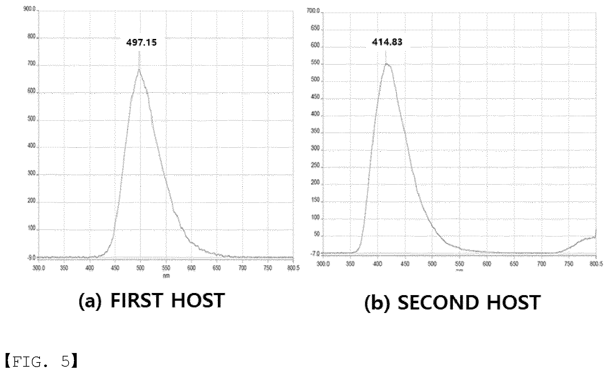

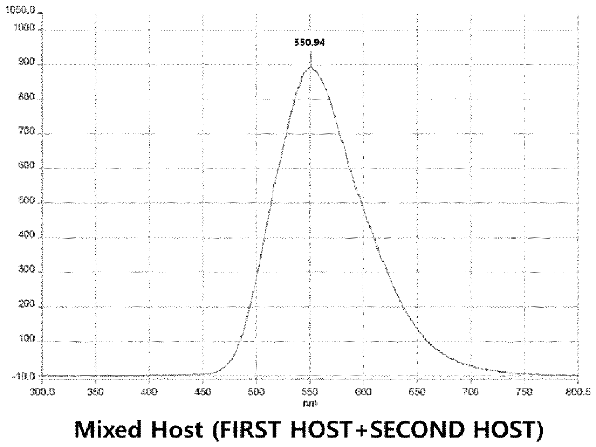

Particularly, the heterocyclic compound of Chemical Formula 1 is a bipolar compound and does not have a strong acceptor ability, however, by injecting an acceptor (n-host) that is the heterocyclic compound corresponding to any one of Chemical Formulae 4 to 6 having a favorable electron transfer ability therewith, a red-shifted change is resulted in the PL (photoluminescence) as shown in FIG. 4 and FIG. 5, and as a result, the exciplex may be formed, which may help with enhancement in the light emitting properties. FIG. 4(a) and FIG. 4(b) are graphs showing changes in the PL (photoluminescence) when using the heterocyclic compound of Chemical Formula 1 as a single host of the organic light emitting device and when using the heterocyclic compound corresponding to any one of Chemical Formulae 4 to 6 as a single host of the organic light emitting device, and FIG. 5 is a graph showing a change in the PL (photoluminescence) when using the heterocyclic compound of Chemical Formula 1 and the heterocyclic compound corresponding to any one of Chemical Formulae 4 to 6 as a mixed host.

In addition, it was identified that, by injecting an acceptor (n-host) that is the heterocyclic compound corresponding to any one of Chemical Formulae 4 to 6 having a favorable electron transfer ability, the lifetime was significantly improved due to proper movement of the light emitting zone in the light emitting layer.

### <Experimental Example 2> Thermal Stability of Organic Electroluminescent Device

For each of the organic electroluminescent devices manufactured as above, a temperature measurement point (Ts) and a time for evaluation were set as described in the following

Table 13, and based on the temperature measurement point, purity at 50°C, 70°C and 90°C was measured using M7000 of McScience Inc. to evaluate thermal stability of the organic light emitting device.

In addition, based on the temperature measurement point, electroluminescent (EL) properties at 50°C, 70°C and 90°C were measured using M7000 of McScience Inc., and with the measurement results, T90 was measured when standard luminance was 6,000 cd/m² through a lifetime measurement system (M6000) manufactured by McScience Inc. in order to measure driving voltage, efficiency and lifetime (T90) of the device.

The results are as shown in the following Table 13 and Table 14.

**[Table 13]**

| Compo und | Ts | Time for Evaluati on | Initial Purity | Ts+50°C | | Ts+70°C | | Ts+90°C | |
|---|---|---|---|---|---|---|---|---|---|
| | | | | Tempera ture | Purit y | Tempera ture | Purit y | Tempera ture | Purit y |
| 11 | 290 | 200 hr | 99.95 | 340 | 99.90 | 360 | 99.78 | 380 | 96.55 |
| 12 | 260 | 200 hr | 99.98 | 310 | 99.89 | 330 | 99.02 | 350 | 94.80 |
| 51 | 290 | 200 hr | 99.93 | 340 | 99.93 | 360 | 99.93 | 380 | 98.88 |
| 52 | 320 | 200 hr | 99.99 | 370 | 99.98 | 390 | 99.97 | 410 | 99.90 |
| 88 | 250 | 200 hr | 99.91 | 300 | 99.91 | 320 | 99.90 | 340 | 98.86 |
| 89 | 280 | 200 hr | 99.99 | 330 | 99.99 | 350 | 99.98 | 370 | 99.90 |
| 123 | 300 | 200 hr | 99.92 | 350 | 99.86 | 370 | 99.75 | 390 | 98.99 |
| 124 | 270 | 200 hr | 99.93 | 320 | 99.86 | 340 | 99.30 | 360 | 97.04 |

**[Table 14]**

| Compoun d | Ts | Driving Voltage (V) | Efficiency (cd/A) | Lifetime (T90) |
|---|---|---|---|---|
| 11 | Ts°C (290°C) | 3.91 | 28.7 | 100 |
| | Ts+50°C (340°C) | 3.91 | 28.6 | 88 |
| | Ts+70°C (360°C) | 3.92 | 28.9 | 50 |
| 12 | Ts°C (260°C) | 3.85 | 20.1 | 95 |
| | Ts+50°C (310°C) | 3.85 | 19.8 | 85 |
| | Ts+70°C (330°C) | 3.87 | 19.8 | 48 |
| 51 | Ts°C (290°C) | 4.10 | 21.6 | 88 |
| | Ts+50°C (340°C) | 4.10 | 22.3 | 76 |
| | Ts+70°C (360°C) | 4.10 | 21.1 | 64 |
| 52 | Ts°C (320°C) | 4.02 | 26.5 | 96 |
| | Ts+50°C (370°C) | 4.02 | 26.5 | 89 |
| | Ts+70°C (390°C) | 4.02 | 26.5 | 70 |
| 88 | Ts°C (250°C) | 4.09 | 21.9 | 103 |
| | Ts+50°C (300°C) | 4.09 | 21.8 | 95 |
| | Ts+70°C (320°C) | 4.10 | 21.5 | 76 |
| 89 | Ts°C (280°C) | 3.95 | 24.6 | 113 |
| | Ts+50°C (330°C) | 3.95 | 24.6 | 100 |
| | Ts+70°C (350°C) | 3.96 | 24.4 | 80 |
| 123 | Ts°C (300°C) | 4.08 | 24.6 | 104 |
| | Ts+50°C (350°C) | 4.08 | 24.5 | 89 |
| | Ts+70°C (370°C) | 4.10 | 24.2 | 65 |
| 124 | Ts°C (270°C) | 3.96 | 20.0 | 94 |
| | Ts+50°C (320°C) | 3.96 | 19.8 | 78 |
| | Ts+70°C (340°C) | 3.97 | 19.8 | 61 |

As seen from Table 13 and Table 14, the compounds of the present disclosure have superior thermal stability due to their structural stability. Particularly, compared to Compounds 11 and 12 according to the present application having a dimethylfluorene group vulnerable to heat in the arylamine functional group, thermal stability is more superior when having phenyl or heterocyclic compound. In addition, it was identified that the molecular weight increased and structural planarity increased when the arylamine functional group did not directly bond and an intermediate phenyl linker was inserted, and as a result, a compound having more superior thermal stability was able to be obtained.

### [Reference Numeral]

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

## Claims

1. A heterocyclic compound represented by the following Chemical Formula 1: 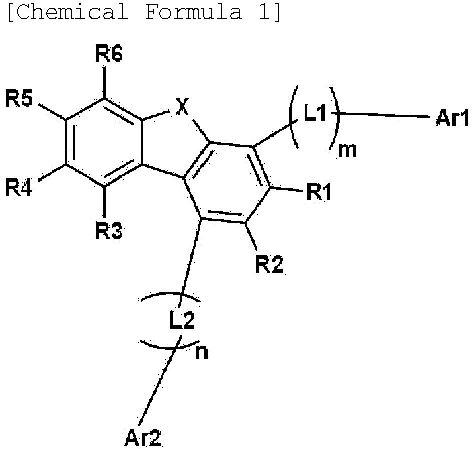
wherein, in Chemical Formula 1,
X is O; or S;
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms;
R1 to R6 are the same as or different from each other, and each independently hydrogen; deuterium; a substituted or unsubstituted aryl group having 6 to 60 carbon atoms; or a substituted or unsubstituted heteroaryl group having 2 to 60 carbon atoms;
Ar1 and Ar2 are the same as or different from each other, and each independently a monocyclic or polycyclic heterocyclic group having 2 to 60 carbon atoms substituted or unsubstituted and comprising one or more 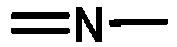 bonds; or a substituted or unsubstituted amine group; and
m and n are each an integer of 0 to 3, and when m and n are each 2 or greater, substituents in the parentheses are the same as or different from each other.

2. The heterocyclic compound of Claim 1, wherein the substituted or unsubstituted means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; - P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

3. The heterocyclic compound of Claim 1, wherein Chemical Formula 1 is represented by the following Chemical Formula 2 or 3: 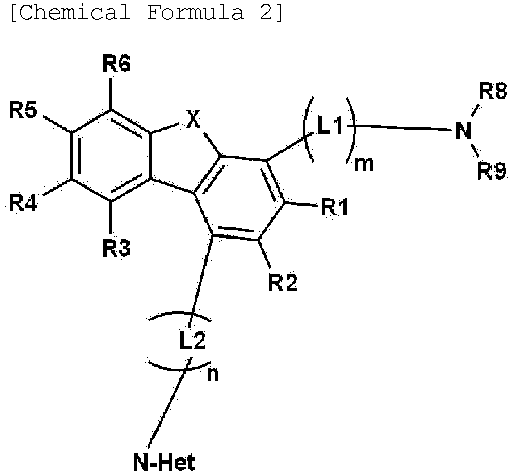 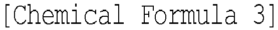 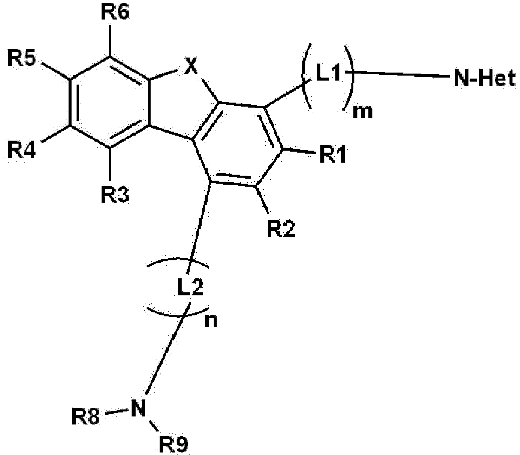
in Chemical Formulae 2 and 3,
R8 and R9 are the same as or different from each other, and each independently hydrogen; deuterium; a substituted or unsubstituted aryl group having 6 to 60 carbon atoms; or a substituted or unsubstituted heteroaryl group having 2 to 60 carbon atoms;
N-Het is a monocyclic or polycyclic heterocyclic group having 2 to 60 carbon atoms substituted or unsubstituted and comprising one or more 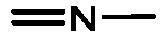 bonds; and
X, L1, L2, R1 to R6, Ar1, Ar2, m and n have the same definitions as in Chemical Formula 1.

4. The heterocyclic compound of Claim 1, wherein N-Het is a group represented by any one of the following Chemical Formulae A-1 to A-3: 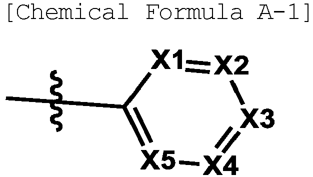 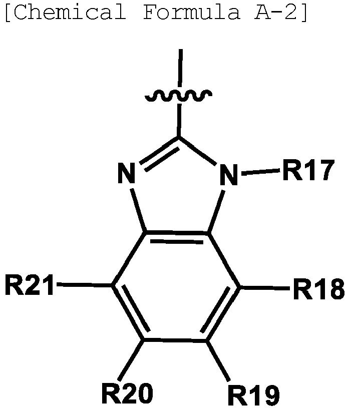 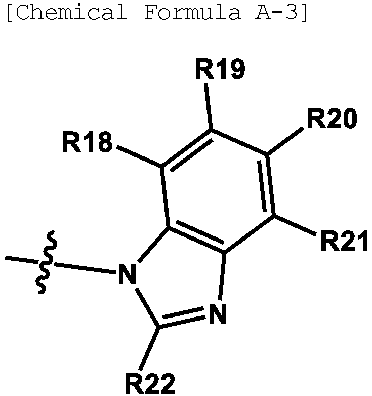 in Chemical Formulae A-1 to A-3,
X1 is CR11 or N, X2 is CR12 or N, X3 is CR13 or N, X4 is CR14 or N, X5 is CR15 or N, and at least one of X1 to X5 is N; and
R11 to R15 and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring, and 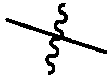 is a site linked to Chemical Formula 1.

5. The heterocyclic compound of Claim 1, wherein R1 to R6 are hydrogen; or deuterium.

6. The heterocyclic compound of Claim 1, wherein at least one of Ar1 and Ar2 is a substituted or unsubstituted amine group.

7. The heterocyclic compound of Claim 1, wherein Chemical Formula 1 is represented by any one of the following compounds:

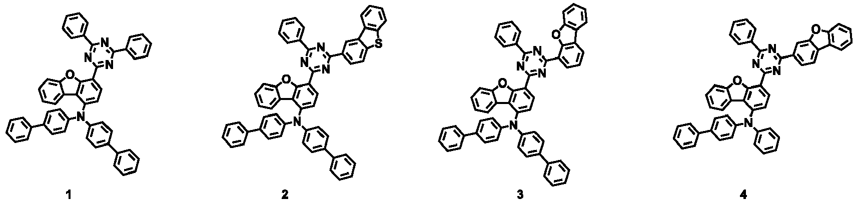

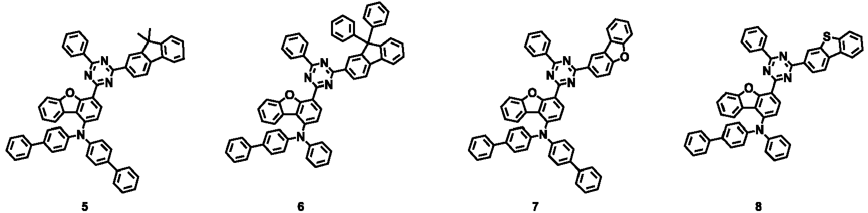

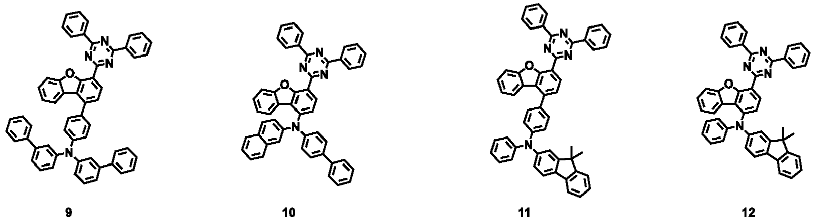

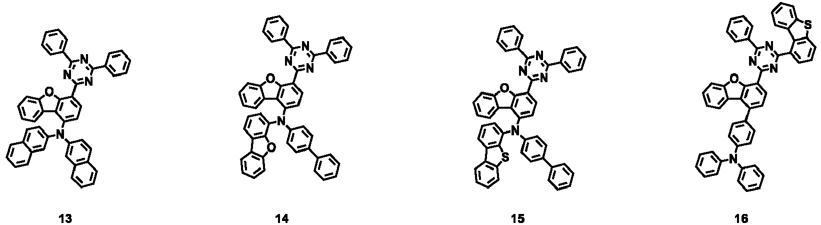

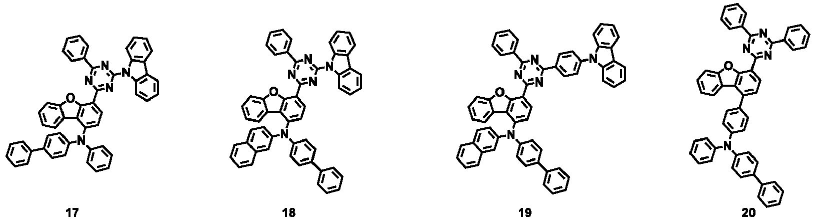

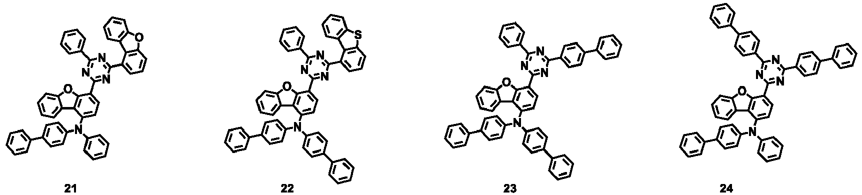

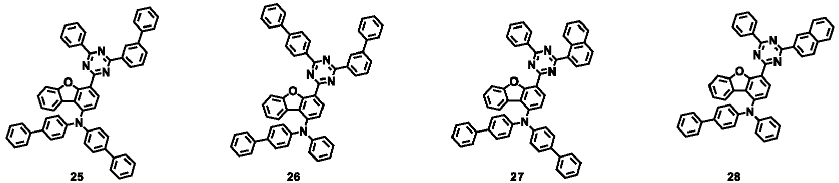

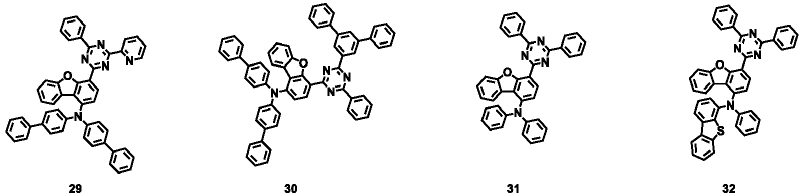

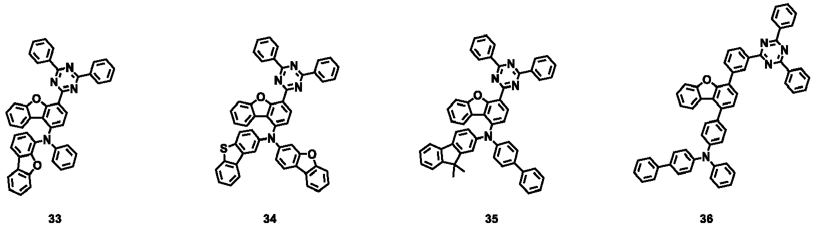

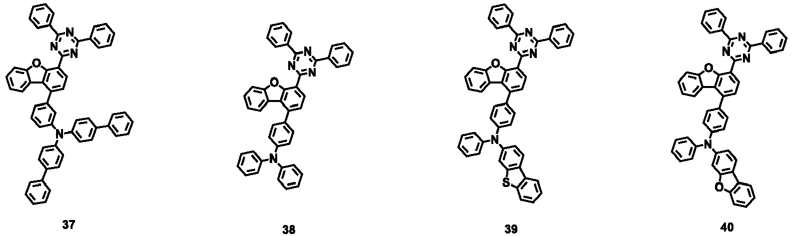

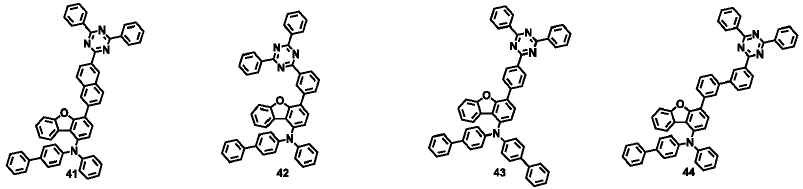

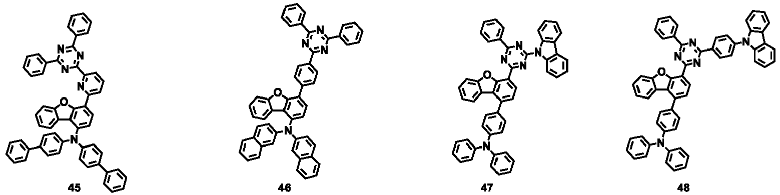

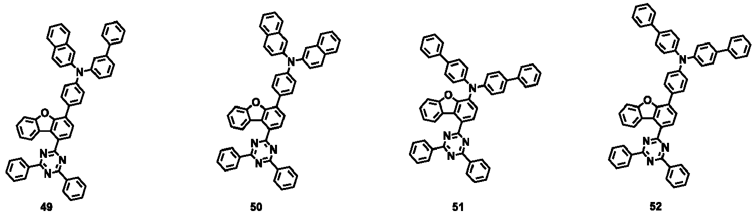

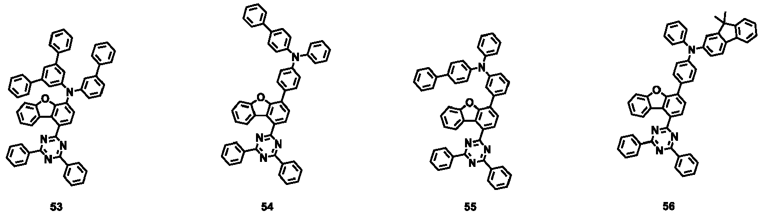

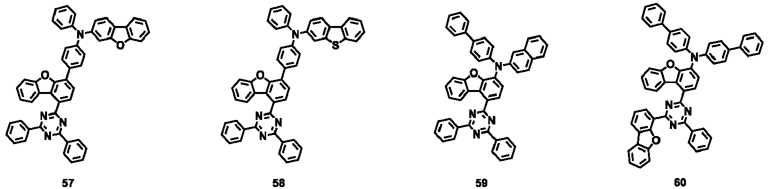

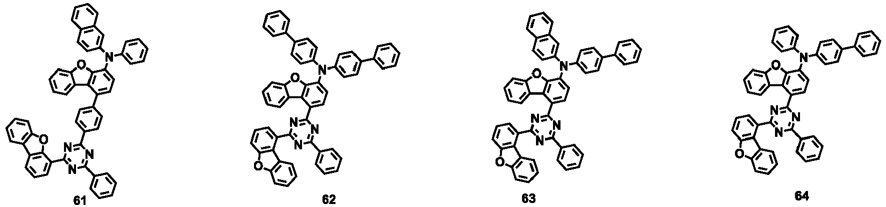

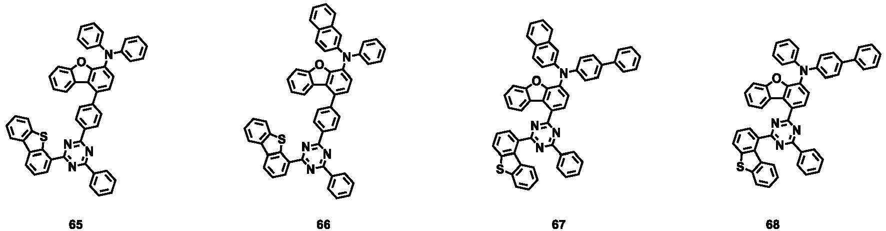

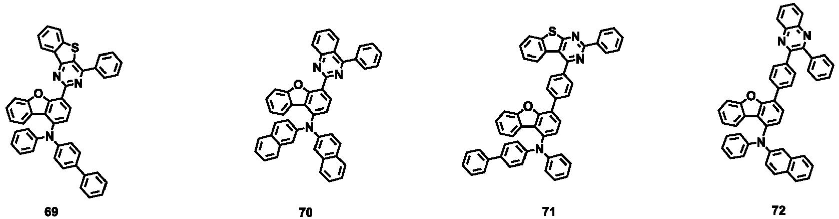

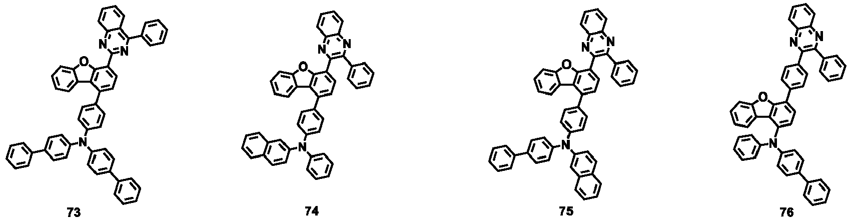

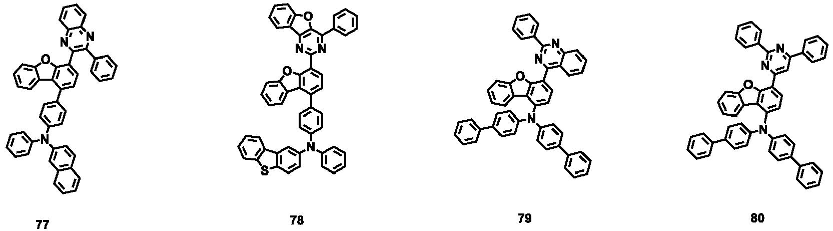

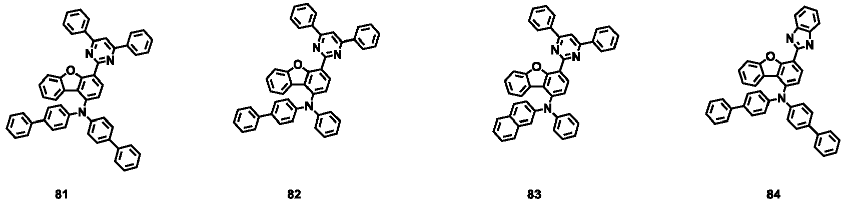

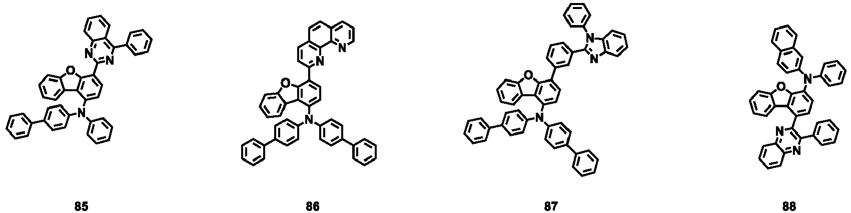

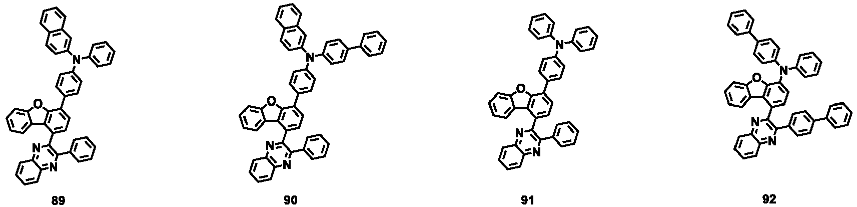

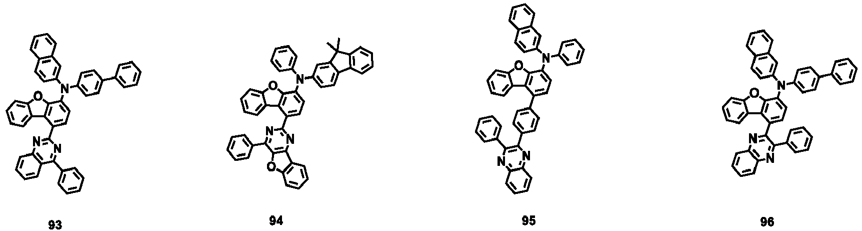

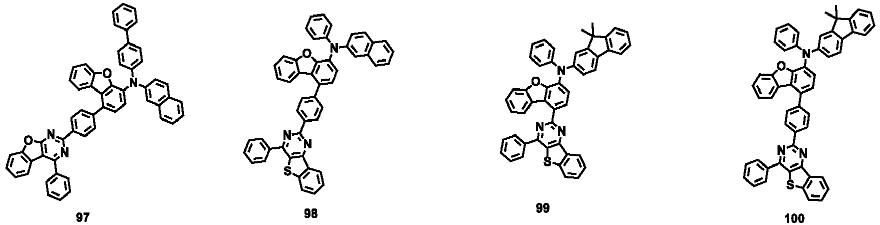

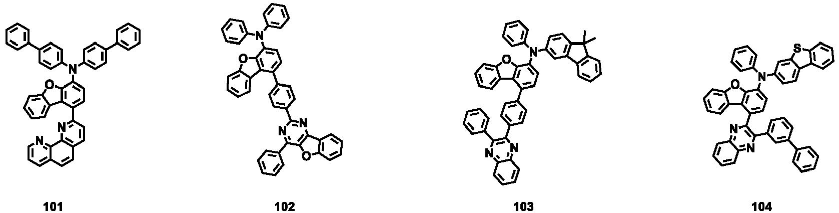

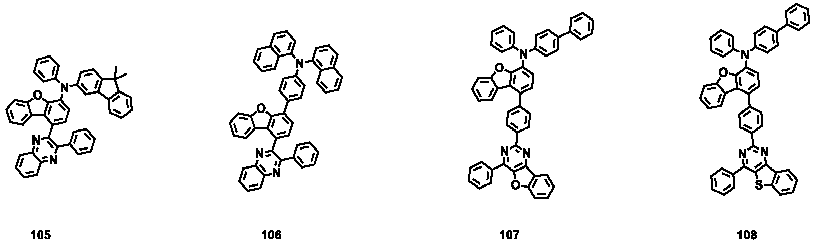

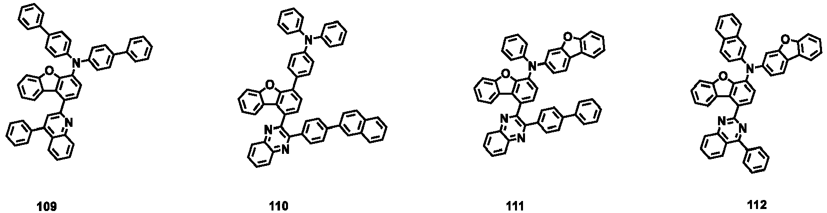

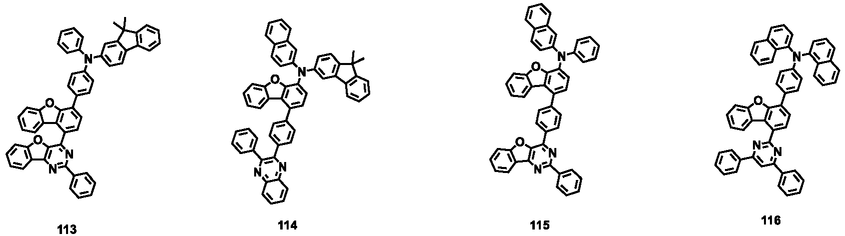

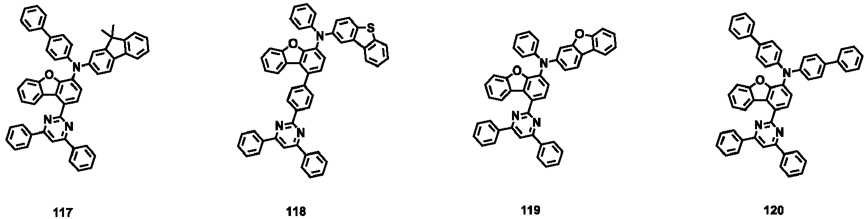

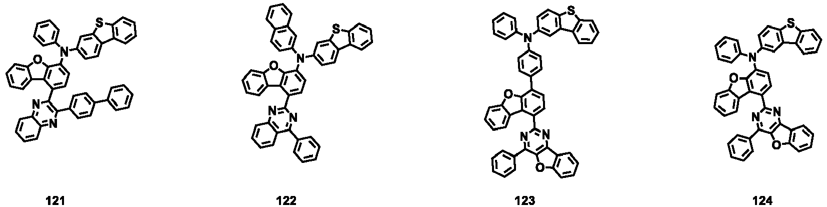

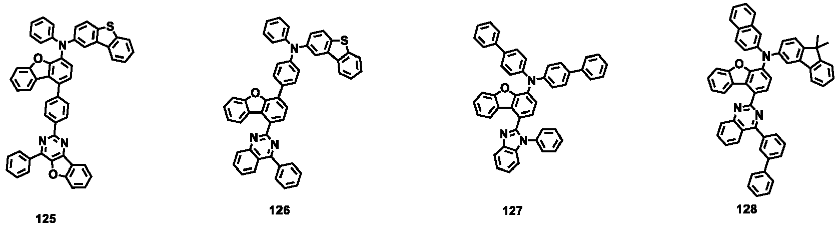

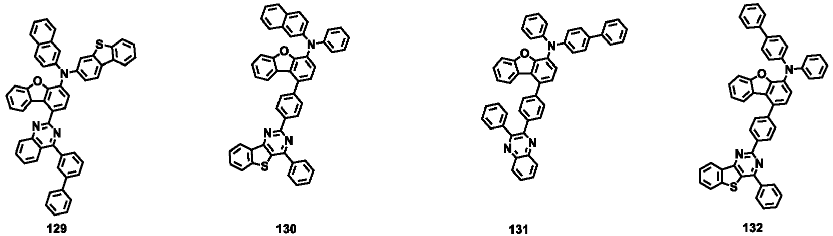

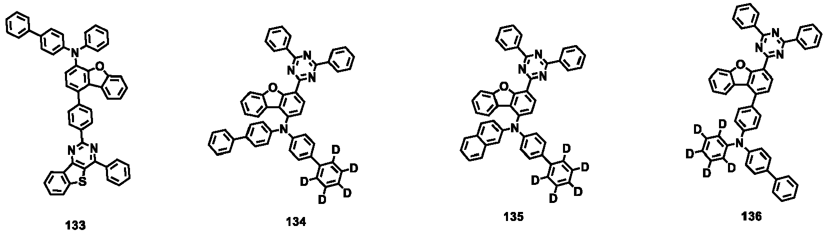

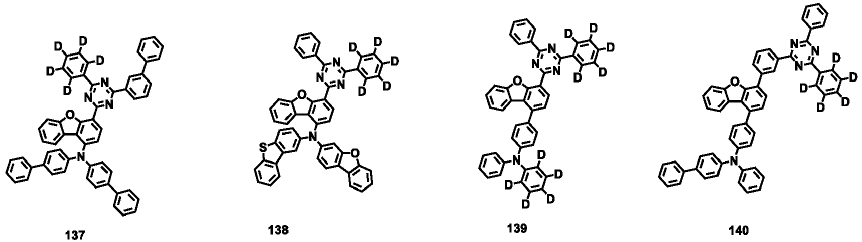

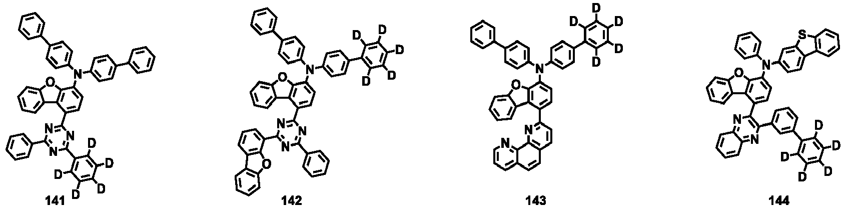

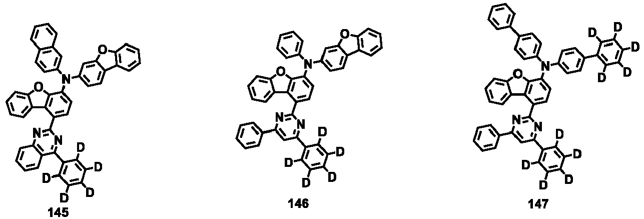

8. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 7.

9. The organic light emitting device of Claim 8, wherein the organic material layer comprises one or more light emitting layers, and the light emitting layer comprises the heterocyclic compound.

10. The organic light emitting device of Claim 9, wherein the light emitting layer comprises two or more host materials, and at least one of the host materials comprises the heterocyclic compound as a host material of a light emitting material.

11. The organic light emitting device of Claim 8, further comprising one, two or more layers selected from the group consisting of a light emitting layer, a hole injection layer, a hole transfer layer, an electron injection layer, an electron transfer layer, a hole auxiliary layer and a hole blocking layer.

12. The organic light emitting device of Claim 8, wherein the organic material layer comprises the heterocyclic compound represented by Chemical Formula 1 as a first compound, and further comprises one of heterocyclic compounds represented by the following Chemical Formulae 4 to 6 as a second compound: 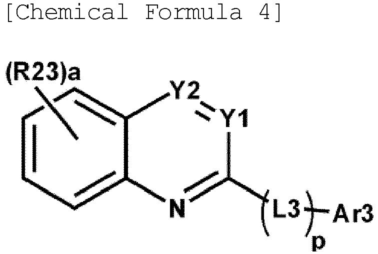 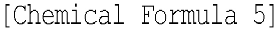 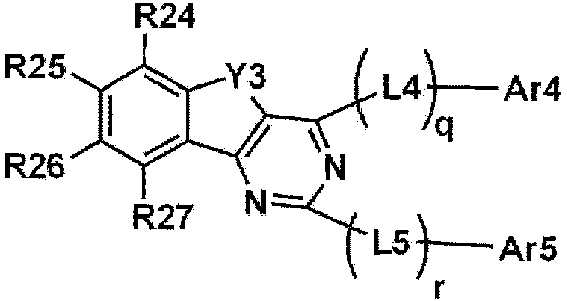 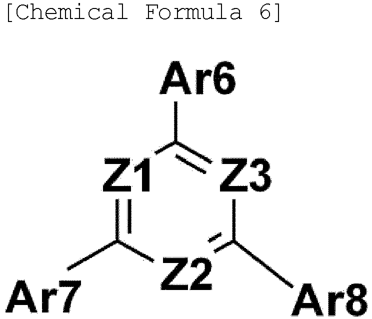 in Chemical Formulae 4 to 6,
L3 to L5 are the same as or different from each other, and each independently a direct bond; a substituted or unsubstituted arylene group having 6 to 60 carbon atoms; or a substituted or unsubstituted heteroarylene group having 2 to 60 carbon atoms;
R23 to R27 are the same as or different from each other, and each independently hydrogen; deuterium; a substituted or unsubstituted aryl group having 6 to 60 carbon atoms; or a substituted or unsubstituted heteroaryl group having 2 to 60 carbon atoms;
Y3 is O; or S;
Ar3 to Ar8 are the same as or different from each other, and each independently a substituted or unsubstituted aryl group having 6 to 60 carbon atoms; or a substituted or unsubstituted heteroaryl group having 2 to 60 carbon atoms;
one of Y1 and Y2 is N, the other one is CRk, and Rk is a substituted or unsubstituted aryl group having 6 to 60 carbon atoms; or a substituted or unsubstituted heteroaryl group having 2 to 60 carbon atoms;
Z1 to Z3 are each independently CH; or N, and at least one of Z1 to Z3 is N;
p, q and r are each an integer of 0 to 3, and when p, q and r are each 2 or greater, substituents in the parentheses are the same as or different from each other; and
a is an integer of 0 to 4, and when a is 2 or greater, substituents in the parentheses are the same as or different from each other.

13. The organic light emitting device of Claim 12, wherein Chemical Formulae 4 to 6 are represented by any one of the following compounds:

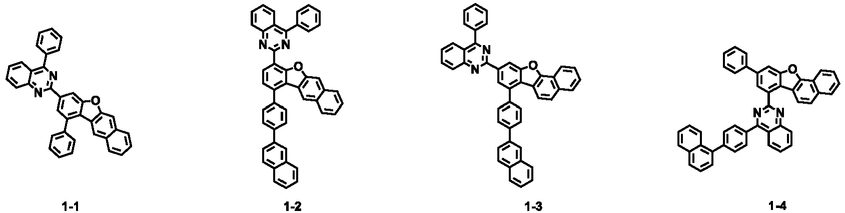

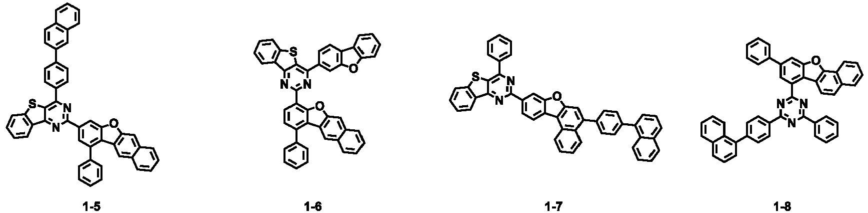

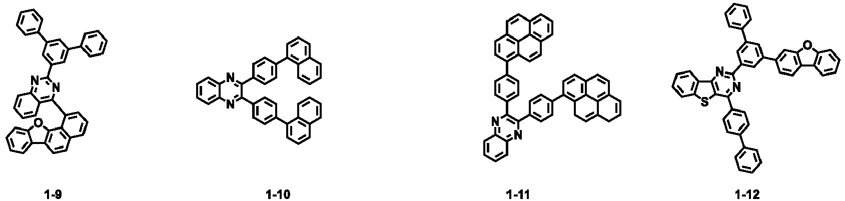

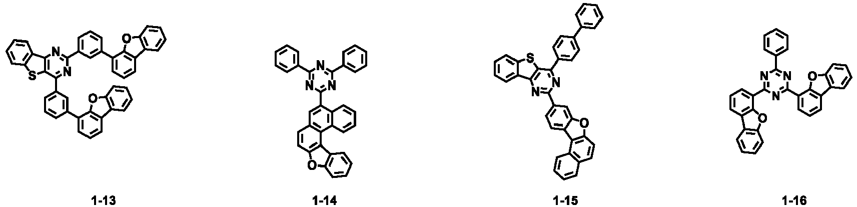

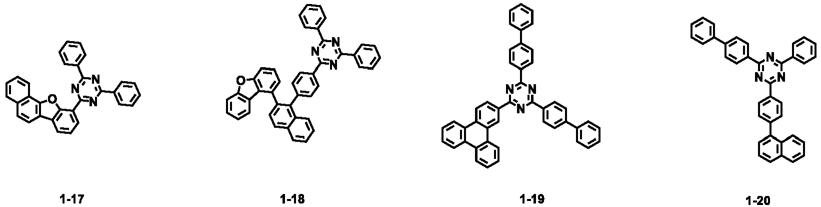

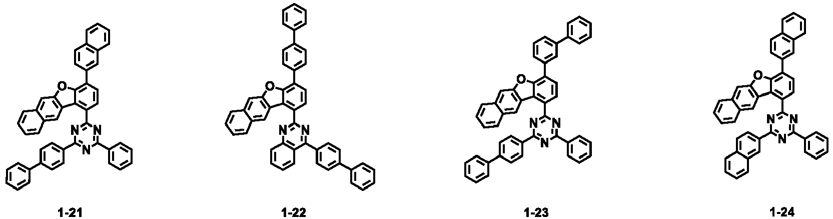

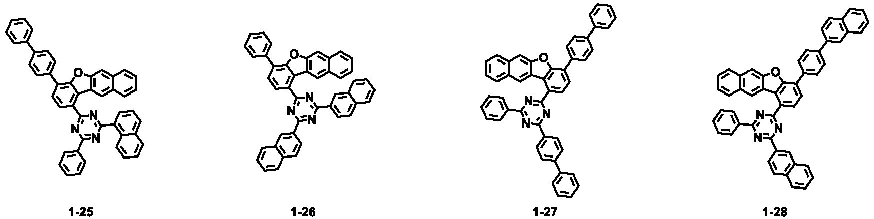

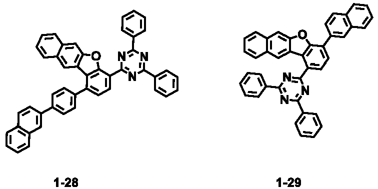

14. A composition for an organic material layer of an organic light emitting device, the composition comprising:
the heterocyclic compound represented by Chemical Formula 1 of Claim 1; and
one of heterocyclic compounds represented by the following Chemical Formulae 4 to 6: 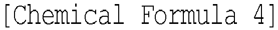 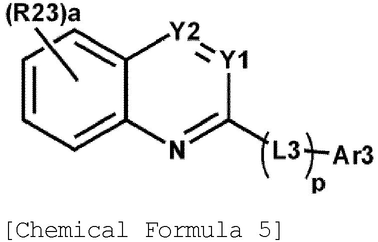 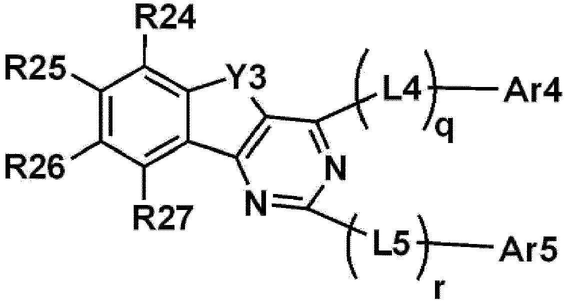 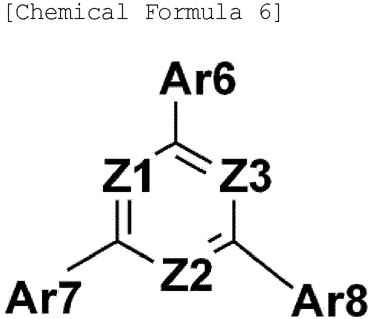
wherein, in Chemical Formulae 4 to 6,
L3 to L5 are the same as or different from each other, and each independently a direct bond; a substituted or unsubstituted arylene group having 6 to 60 carbon atoms; or a substituted or unsubstituted heteroarylene group having 2 to 60 carbon atoms;
R23 to R27 are the same as or different from each other, and each independently hydrogen; deuterium; a substituted or unsubstituted aryl group having 6 to 60 carbon atoms; or a substituted or unsubstituted heteroaryl group having 2 to 60 carbon atoms;
Y3 is O; or S;
Ar3 to Ar8 are the same as or different from each other, and each independently a substituted or unsubstituted aryl group having 6 to 60 carbon atoms; or a substituted or unsubstituted heteroaryl group having 2 to 60 carbon atoms;
one of Y1 and Y2 is N, the other one is CRk, and Rk is a substituted or unsubstituted aryl group having 6 to 60 carbon atoms; or a substituted or unsubstituted heteroaryl group having 2 to 60 carbon atoms;
Z1 to Z3 are each independently CH; or N, and at least one of Z1 to Z3 is N;
p, q and r are each an integer of 0 to 3, and when p, q and r are each 2 or greater, substituents in the parentheses are the same as or different from each other; and
a is an integer of 0 to 4, and when a is 2 or greater, substituents in the parentheses are the same as or different from each other.

15. The composition for an organic material layer of an organic light emitting device of Claim 14, wherein, in the composition, the heterocyclic compound represented by Chemical Formula 1:the heterocyclic compound represented by any one of Chemical Formulae 4 to 6 have a weight ratio of 1:10 to 10:1.
